(19) Europäisches Patentamt
European Patent Office
Office européen des brevets

(11) **EP 3 136 988 B1**

(12) **EUROPÄISCHE PATENTSCHRIFT**

(45) Veröffentlichungstag und Bekanntmachung des Hinweises auf die Patenterteilung:
**02.10.2019 Patentblatt 2019/40**

(21) Anmeldenummer: **15704506.3**

(22) Anmeldetag: **05.02.2015**

(51) Int Cl.:
***A61B 17/128*** (2006.01)

(86) Internationale Anmeldenummer:
**PCT/EP2015/052443**

(87) Internationale Veröffentlichungsnummer:
**WO 2015/165602 (05.11.2015 Gazette 2015/44)**

(54) **MEDIZINISCHES SCHAFTINSTRUMENT MIT UNTERSCHIEDLICHEN LAGERPOSITIONSABSTÄNDEN DURCH KLAMMER-MITNAHMEELEMENTE UND RÜCKHALTELASCHEN FÜR KLAMMERN**

MEDICAL SHAFT-TYPE INSTRUMENT WITH DIFFERENT STORAGE POSITION DISTANCES DUE TO CLAMP ENTRAINING ELEMENTS AND RETAINING LUGS FOR CLAMPS

INSTRUMENT MÉDICAL À TIGE PRÉSENTANT DIFFÉRENTS ÉCARTS DE POSITION DE STOCKAGE ENTRE DES ÉLÉMENTS D'ENTRAÎNEMENT DE CLAMPS ET DES LAMES DE RETENUE POUR CLAMPS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.04.2014 DE 102014207900**
**28.04.2014 DE 102014207955**
**28.04.2014 DE 102014207971**

(43) Veröffentlichungstag der Anmeldung:
**08.03.2017 Patentblatt 2017/10**

(73) Patentinhaber: **Aesculap AG**
**78532 Tuttlingen (DE)**

(72) Erfinder:
• **SCHOLTEN, Thomas**
**78532 Tuttlingen (DE)**
• **WANKE, Gunnar**
**8280 Kreuzlingen (CH)**

• **TIMMERMANN, Jörg Hinrich**
**78573 Wurmlingen (DE)**
• **BENK, Michael**
**78573 Wurmlingen (DE)**
• **WURSTHORN, Rainer**
**78052 VS-Obereschach (DE)**

(74) Vertreter: **Winter, Brandl, Fürniss, Hübner, Röss, Kaiser, Polte - Partnerschaft mbB Patent- und Rechtsanwaltskanzlei Alois-Steinecker-Strasse 22 85354 Freising (DE)**

(56) Entgegenhaltungen:
EP-A1- 0 089 737        EP-A1- 0 409 569
EP-A1- 2 158 855        EP-A1- 2 609 877
EP-A2- 0 086 721        WO-A1-03/005911
DE-A1-102009 018 820

**Beschreibung**

Die Erfindung betrifft ein medizinisches Schaftinstrument, vorzugsweise der Minimalinvasiv-Bauart, mit einem Instrumentenkopf / Maulteil zum Applizieren von Klammern, etwa Ligaturklammern, Clips, Klipsen oder (Feder-)Klemmen, also solchen Bauteilen, die nach einer "plastisch/elastischen" Verformung abklemmend wirken, wobei der Instrumentenkopf über einen Instrumentenschaft mit einem Instrumentengriff, wie einem "Challenger-Griff", zur Betätigung / Aktuierung des Instrumentenkopfs verbindbar ist, und mit einem ein Gehäuse aufweisenden Clipmagazin, in welchem eine Vielzahl der Klammern nach dem Einlagerungsprinzip / Einzellagerungsprinzip mit einem vorbestimmten Lagerpositionsabstand zueinander gebunkert sind, welche sämtlich mittels einer hin- und herbewegbaren Förder- und Mitnahmeschiene, etwa nach Art einer Vorschubschiene / eines Vorschubbleches, im Rahmen eines einzigen Förderhubes / Vorschubhubes um jeweils eine Lagerposition / Ruhelage / Ruheposition, also jener Position, in der die jeweilige Klammer im Clipmagazin bei Stillstand der Förder- und Mitnahmeschiene abgelegt ist, vorrückbar sind, wobei die Förder- und Mitnahmeschiene (wenigstens) ein Klammer-Mitnahmeelement aufweist.

Der Instrumentenkopf bildet über ein oberes und ein unteres Maulteil bzw. eine obere und untere Maulteilbranche einen Maulteilbereich aus. Der Instrumentengriff kann als "Challenger"-Griff ausgebildet sein und/oder mit Handkraft und/oder Gasdruck betrieben werden.

Der Begriff "Clip" wird synonym zum Begriff "Klammer" verwendet. Eine Klammer hat einen unverpressten Ausgangszustand und einen verpressten Endzustand. Normalerweise befindet sich im verpressten Zustand Gewebe zwischen zwei zusammengehörenden Klammer- / Cliparmen, bspw. ein Hohlorgan wie ein Blutgefäß. In diesem Fall bestimmen die Dicke und die Konsistenz der Wandung des Blutgefäßes die Breite des Spalts zwischen den Klammer- / Cliparmen im verpressten Zustand.

Aus dem Stand der Technik sind bereits medizinische Schaftinstrumente bekannt, wie etwa aus der EP 0 697 198 A1. Dort wird ein chirurgisches Anlegegerät für Clipse offenbart. Um bei einem chirurgischen Anlegegerät für U-förmige Clipse mit einem Griff, einem daran anschließenden Rohrschaft, mit einem zangenförmigen Anlegewerkzeug am freien Ende des Rohrschafts und einem Clips-Magazin im Rohrschaft, mit einem vom Griff aus betätigbaren, im Rohrschaft angeordneten Schließmechanismus für das Anlegewerkzeug und mit einem ebenfalls vom Griff aus betätigbaren, im Rohrschaft angeordneten Vorschubmechanismus für die Clipse eine Wiederverwendung der wesentlichen Teile des Anlegegeräts zu ermöglichen, wird dort vorgeschlagen, dass der Rohrschaft eine seitliche Durchbrechung aufweist, in die das Clips-Magazin von außen lösbar derart einsetzbar ist, dass es mit dem Vorschubmechanismus in Wirkverbindung tritt und dass der Auslass des Clips-Magazins mit einem die Clipse in das Anlegewerkzeug führende Vorschubbahn ausgerichtet ist.

Ein solches Clips-Magazin ist auch aus der DE 10 2009 018 820 A1 bekannt. Um bei einem Magazin mit einer Vielzahl C-förmiger Ligaturklammern, mit einem die in einer Reihe hintereinander und parallel zueinander angeordneten Ligaturklammern aufnehmenden Gehäuse, mit einem relativ zu dem Gehäuse in Richtung der Reihe vor- und zurückschiebbaren Transportelemente, welches beim Vor- und Zurückschieben relativ zum Gehäuse einen Vorschub mindestens einer Ligaturklammer in Richtung auf ein Austrittsende des Magazins bewirkt, den Aufbau des Magazins zu vereinfachen und die Baugröße zu reduzieren, wird dort vorgeschlagen, dass die Ligaturklammern jeweils zwei über einen Brückenabschnitt verbundene Schenkel aufweisen und durch einen Längsschlitz in zwei nebeneinander liegende Abschnitte unterteilt sind, die im Bereich der freien Enden der Schenkel miteinander verbunden sind, und dass das Transportelement die in einer Reihe angeordneten Ligaturklammern in den Zwischenraum zwischen den beiden nebeneinander liegenden Abschnitten der Ligaturklammer durchsetzt.

Diese bestehenden Vorrichtungen setzen also chirurgische Klammern oder Clips ein, die im Grundsatz auch schon bekannt sind.

So vertreibt die Firma Applied Medical einen chirurgischen Clip EPIX Universal CA500, bei dem die beiden im Wesentlichen geraden Klammerstege / Schenkel / Cliparme über einen im Wesentlichen V-förmigen Abschnitt miteinander verbunden sind. Die beiden geraden Cliparme verlaufen im Wesentlichen parallel zur Längsachse des chirurgischen Clips und in den Übergangsbereichen zwischen den Cliparmen und dem Verbindungsabschnitt sowie an der Kehle des Verbindungsabschnitts sind relativ kleine Biegeradien ausgebildet. Dies bedeutet, dass die Übergangsbereiche durch Knicke gebildet sind. Vergleichbare chirurgische Clips mit vergleichbarer Geometrie werden auch von United States Surgical unter dem Namen Endo Clip Autosuture 5 mm und von Ethicon unter dem Namen Ligamax 5 vertrieben. Eine etwas andere Geometrie weist der Endo Clip Autosuture III 5 mm auf, der ebenfalls von United States Surgical vertrieben wird. Dieser Clip weist ebenfalls zwei im Wesentlichen gerade, parallele Cliparme sowie einen Verbindungsabschnitt für die beiden geraden Cliparme auf, der mit einer Kehle ausgebildet ist, welche einen recht kleinen Krümmungsradius aufweist. Im Gegensatz zu den vorbeschriebenen chirurgischen Clips sind die Übergangsbereiche zwischen den Cliparmen und dem Verbindungsabschnitt mit einem deutlich größeren Krümmungsradius ausgebildet, also eher als gebogene Abschnitte denn als geknickte Abschnitte. Die Patentanmeldung US 2011/0224701 A1 offenbart einen chirurgischen Clip mit einer halbkreisförmigen Außenfläche und einer profilierten Innenfläche. Die halbkreisförmige Außenfläche dient dazu, ein Verkeilen des Clips im Maulteil eines Clipapplikators zu verhindern und die profilierte Innenfläche soll die Haftung auf dem geklemmten Gewebe verbessern. Die Cliparme des Clips weisen in einer Seitenansicht jeweils gerade Abschnitte auf, welche im Bereich des distalen Endes des Clips, also an seiner offenen Seite, parallel zueinander verlaufen. Die Cliparme bestehen zudem aus mehreren im Wesentlichen unverformbaren Abschnitten, welche durch verformbare Abschnitte verbunden sind. Allen bekannten Clips ist gemeinsam, dass jeder Cliparm einen im Wesentlichen geraden Abschnitt aufweist und der Verbindungsabschnitt zwei im Wesentlichen gerade Abschnitte aufweist. In der Kehle sowie im Übergangsbereich zwischen Cliparm und Verbindungsabschnitt sind dann mehr oder weniger gekrümmte Abschnitte ausgebildet. All diese Clips sind zudem Einfachstegclips, d.h. Clips, die aus einem Stück Draht gebogen werden können und sich im Wesentlichen in einer Ebene erstrecken (abgesehen von einer Drahtsicke).

Einfachstegclips weisen immer zwei Cliparme auf. Ein Problem dieser Art von Clips ist, dass sie beim Applizieren, d.h. beim Verpressen durch einen Clipapplikator, teilweise ungünstige Eigenschaften haben. Zum einen wird durch die Ausbildung des recht geringen Krümmungsradius im Übergangsbereich von Cliparm zu Verbindungsabschnitt ein Bereich geschaffen, in dem das Material des Clips (Metall, beispielsweise Titan oder Titanlegierungen) mehr gestreckt wird als in den angrenzenden Bereichen, was dazu führt, dass dieser Bereich, im Folgenden Knick genannt, beim Verpressen des Clips mit einem Applikator nicht vollständig in eine gerade Form zurückverformt werden kann. Es verbleibt somit ein Stelle in dem verpressten Clip, an dem die beiden Cliparme weiter beabstandet sind. Dies führt zu einem nicht optimalen Verschluss des jeweils geclipsten, d.h. zusammengedrückten Gefäßes. Ein weiteres Problem dieser Art von Clips ist, dass der Verschluss dieser Clips im verpressten Zustand an den distalen Enden der Cliparme schwach ist. Dies bedeutet, dass an den distalen Clipenden kaum mehr eine Kraft auf das Gefäß aufgebracht wird, welche zu einem Verschluss des Gefäßes führt. Während des Verpressvorgangs werden die beiden parallelen Cliparme um die Kehle des Clips herum nach innen verformt. Dabei verbleibt nur der Knick bzw. der Übergangsbereich des Clips in Kontakt mit der jeweiligen Branche des Clipapplikators. Erst wenn die distalen Enden des Clips sich berühren (falls kein Gewebe gegriffen wird) oder beiderseits des zu greifenden Gewebes in Anlage kommen (wenn Gewebe gegriffen wird), wird der Knick zwischen dem Cliparm und dem Verbindungsabschnitt aufgebogen. Dabei verformen sich die distalen Enden der Cliparme nach außen und der maßgebliche Krafteinleitungspunkt in das Gewebe verlagert sich zu dem Knick hin. Dies entlastet wiederum die distalen Enden der Cliparme und sie entspannen ihre elastische Verformung (im Wesentlichen unter Beibehaltung ihrer aktuellen Lage). Wenn nun der Knick in dem Cliparm weitest möglich zurückverformt wurde, der Clip also vollständig verpresst ist, führt dies dazu, dass der Clip an den distalen Enden der Cliparme kaum mehr eine Schließkraft bzw. Verpresskraft aufbringen kann, da sich die distalen Enden der Cliparme leicht elastisch nach außen verformen lassen und da es im mittleren Bereich des Clips Stellen gibt, an denen sich die Cliparme berühren (falls keine Gewebe gegriffen wird) oder die Cliparme beiderseits des gegriffenen Gewebes im Wesentlichen punktuell anliegen (dieser Bereich ist im Folgenden in beiden Fällen als mittlerer Kontaktbereich bezeichnet). Auf diese Weise sind die distalen Enden nämlich gegen Ende des Verpressvorgangs im Wesentlichen vollständig entlastet worden, als der Knick in dem Cliparm mit einer Kraft zurückverformt wurde, die jene Kraft bei weitem überschreitet, welche erforderlich ist, um die übrigen Bereiche des Clips in die verpresste Form des Clips zu bringen. Die Verpresskraft, die eigentlich möglichst gleichmäßig über die Länge des verpressten Clips verteilt sein sollte, konzentriert sich dann auf die Nähe der Clipkehle sowie den mittleren Kontaktbereich des Clips.

Gattungsgemäßer Stand der Technik gemäß dem Oberbegriff des Anspruchs 1 ist überdies in folgendem Dokument offenbart: EP0086721 A2.

Die DE 10 2009 018820 A1, EP 0 409 569 A1, EP 2 609 877 A1, EP 0 089 737 A1 und WO03/005911 A1 offenbaren weiteren Stand der Technik aus dem Gebiet der medizinischen Instrumente zum Applizieren von Klammern.

Die Aufgabe einer parallelen Patentanmeldung ist es daher, einen chirurgischen Clip zu schaffen, bei dem sich ein gleichmäßiger und möglichst kleiner Spalt zwischen den Cliparmen bildet und bei dem die Cliparme bis zu ihrem distalen Ende hin eine ausreichende Verpresskraft liefern. Eine weitere Aufgabe ist es dabei, einen chirurgischen Clip bereit zu stellen, welcher über im Wesentlichen die gesamte Cliplänge eine gleichmäßige Kraft auf das gegriffene Gewebe ausübt. Noch eine weitere Aufgabe ist es dabei, ein Herstellungsverfahren für einen solchen Clip bereit zu stellen.

Hierzu hat die Anmelderin bereits eine Lösung erarbeitet. So wird diese vorstehende Aufgabe bei einem chirurgischen Clip für einen chirurgischen Clipapplikator mit wenigstens einem Paar von Cliparmen, wobei jeder Cliparm ein distales Ende oder proximales Ende aufweist und die beiden Cliparme eines Paares von Cliparmen an ihren proximalen Enden miteinander verbunden sind, so dass sie eine Clipkehle ausbilden, in jener parallelen Erfindung dadurch gelöst, dass ein Tangentenwinkel zwischen einer Tangente an eine neutrale Faser eines Cliparms und einer Senkrechten auf eine Längsachse des Clips über die gesamte Länge des Cliparms im Wesentlichen stetig wächst.

Die hier vorliegende Erfindung betrifft auch ein medizinisches Schaftinstrument mit einem solchen chirurgischen Clip, der auch als Doppelsteg-Clip bezeichnet werden kann. Der Clip kann dabei wie in bereits vorgestellter Weise weitergebildet werden. So ist es für den Clip von Vorteil, wenn der Tangentenwinkel bei einer Länge auf dem Cliparm von 6 % einen Wert von höchstens 40° aufweist, vorzugsweise einen Wert von 30°, und weiter vorzugsweise einen Wert von 20° bis 30°. Der Tangentenwinkel kann bei einer Länge auf den Cliparmen von 15% einen Wert von mindestens 50° aufweisen, vorzugsweise einen Wert von mindestens 58° und weiter vorzugsweise einen Wert von 58° bis 75°.

Auch ist es von Vorteil, wenn der Tangentenwinkel bei einer Länge auf dem Cliparm von 22 % einen Wert von mindestens 60° aufweist, vorzugsweise einen Wert von mindestens 67° und weiter vorzugsweise einen Wert von 67° bis 80°.

Wenn der Tangentenwinkel bei einer Länge auf dem Cliparm von 27% einen Wert von mindestens 65° aufweist, vorzugsweise einen Wert von mindestens 72°, und weiter vorzugsweise einen Wert von 72° bis 85°, kann eine vorteilhafte Clipausgestaltung ereicht werden.

Der Tangentenwinkel kann bei einer Länge auf dem Cliparm von 100 % einen Wert von höchstens 88° aufweisen, vorzugsweise einen Wert von 80° bis 88°. Es ist dabei von Vorteil, wenn eine erste Ableitung der Krümmung einer neutralen Faser eines Cliparms im Bereich einer Länge auf dem Cliparm von 8 % bis 100 % keinen Vorzeichenwechsel aufweist, weiter vorzugsweise über die gesamte Länge des Cliparms kein Vorzeichenwechsel erfolgt.

Wenigstens zwei Paare von Cliparmen können vorgesehen sein, wobei ein Cliparm eines Paares von Cliparmen an seinem distalen Ende mit wenigstens einem distalen Ende eines anderen Cliparms eines anderen Paares von Cliparmen verbunden sein kann und einen distalen Verbindungsabschnitt bilden kann. Der Clip kann zwei bzw. drei Paare von Cliparmen aufweisen und der Clip kann als offener oder geschlossener Ringclip oder Doppelringclip ausgebildet sein, vorzugsweise mittels Stanzen, Laser-Sintern, Walzen, Gießen, Metal-Inject-Molding und/oder Schneiden, insbesondere LaserSchneiden oder Wasserstrahlschneiden geschaffen sein.

Im Bereich des distalen Endes eines Cliparms und/oder eines distalen Verbindungsabschnitts kann eine Aussparung vorgesehen sein, die nach proximal hin offen und nach distal hin geschlossen ist, so dass die Aussparung von proximal zugänglich ist und eine erste Anlagefläche nach distal bildet und vorzugsweise eine zweite Anlagefläche nach lateral oder medial bildet, wobei die Aussparung weiter vorzugsweise mittels Stanzen und/oder Prägen hergestellt ist. Die Aussparung kann in einem distalen Verbindungsbereich zweier distaler verbundener Cliparme ausgebildet sein, vorzugsweise, indem die Querschnittsabmessungen des distalen Verbindungsabschnittes auf der proximalen und/oder der medialen Seite des Cliparms ausgebildet ist, wobei eine Reduzierung der Querschnittsabmessung vorzugsweise nach distal und/oder lateral erfolgt, wobei weiter vorzugsweise eine Verjüngung im Wesentlichen auf die halbe Querschnittsabmessung erfolgt.

Es ist auch von Vorteil, wenn ein proximaler Verbindungsbereich zweier Cliparme, ein Cliparm und/oder ggf. ein distaler Verbindungsabschnitt zweier Cliparme eine veränderliche Höhe und/oder Breite in wenigstens einer Richtung quer zur neutralen Phase aufweist, vorzugsweise von mindestens ± 10 %. Es ist zweckmäßig, wenn sich die Querschnittsfläche eines Cliparms und/oder eines proximalen Verbindungsbereichs zu der Clipkehle hin verändert, wobei sich insbesondere die Breite des Cliparms und/oder des proximalen Verbindungsbereichs verändert, insbesondere vergrößert. Dabei kann wenigstens ein Cliparm zumindest einen Abschnitt mit einer Wellenform und/oder einer Zickzack-Form in einer Richtung parallel, quer und/oder senkrecht zur Clipebene aufweisen. Ein Paar von Cliparmen kann von lateral her im Querschnitt veränderte, insbesondere verjüngte Cliparme aufweisen, vorzugsweise durch einen Kantenbruch im distalen Bereich der Cliparme, insbesondere durch einen vergrößerten Radius an der Außenkante des Cliparms. Wenigstens ein Cliparm kann an seiner Innenfläche ein Profil aufweisen, welches vorzugsweise mittels Prägen, Walzen, Schneiden oder mittels Drahterosion oder Senkerosion ausgebildet ist.

Solche chirurgischen Clips können in einer Abfolge von Schritten geschaffen sein, etwa durch Stanzen eines Rohlings aus einem Blech, wobei der Rohling an wenigstens einer Stelle mit dem verbleibenden Blech verbunden bleibt, vorzugsweise im Bereich der späteren Clipkehle, einem nachfolgenden Biegen des Rohlings in einer Richtung quer zum verbleibenden Blech, so dass die Cliparme von der Blechebene vorstehen, wobei dieses Biegen in mehreren Schritten erfolgen kann und nachfolgend ein Trennen von dem verbleibenden Blech erfolgt. Es ist ein Schritt des Prägens eines Profils auf wenigstens eine Seite des Blechs vor und/oder nach dem Stanzen des Rohlings ausführbar, wobei das Prägen in mehreren Schritten ausgeführt werden kann.

Die Cliparme eines Paars von Cliparmen sind üblicherweise aus einem Stück gefertigt und somit stofflich miteinander verbunden. Die Cliparme können prinzipiell aber auch einzeln ausgebildet sein und anschließend bspw. durch Schweißen miteinander verbunden werden. Aus dem Stand der Technik sind auch Maulteile, die synonym auch als Instrumentenköpfe bezeichnet werden können, bekannt.

In der europäischen Patentanmeldung EP 1 712 187 A2 ist ein Instrumentenkopf gezeigt, bei dem die beiden Maulteilbranchen elastisch federnd über eine gemeinsame Basis verbunden sind. Im Bereich ihrer distalen Enden, welche dazu vorgesehen sind, den chirurgischen Clip zu halten und zusammen zu drücken und den Clip dadurch zu applizieren, weisen die beiden Branchen auf ihren Außenseiten je eine Gleitfläche auf. Um den Instrumentenkopf zu schließen und so den Clip zu applizieren, wird der Instrumentenkopf gegenüber dem Schaft, in dem es angeordnet ist, nach proximal verschoben (der Instrumentenkopf wird also teilweise in den Schaft hinein gezogen bzw. der Schaft wird über den Instrumentenkopf geschoben) und der distale Rand des Schafts gleitet an den Gleitflächen ab. Durch die Schrägstellung der Gleitflächen gegenüber der Achse des Schafts werden die distalen Enden der Branchen nach innen gedrängt, während die proximalen Enden der Branchen durch die Basis gehalten werden. Auf diese Weise führen die Branchen jeweils eine Drehbewegung um den Punkt herum aus, an dem die Branchen mit der Basis verbunden sind. Ein Öffnungsvorgang des Instrumentenkopfes erfolgt zudem ohne Führung und wird ausschließlich durch die Elastizität der Branchen gewährleistet, die in ihre Ausgangsposition zurück drängen, wenn der Instrumentenkopf während des Öffnungsvorgangs aus dem Schaft heraus geschoben wird.

Einen vergleichbaren Instrumentenkopf ist auch in der internationalen Patentanmeldung WO 2008/127 968 gezeigt, auch wenn sich das dort gezeigte Instrument insgesamt stark von dem vorstehend beschriebenen Instrument unterscheidet. Noch deutlicher wird die Drehbewegung der Branchen beim Öffnen und Schließen des Instrumentenkopfes aus der US Patentanmeldung US 2005/0171560 A1. Dort sind die distalen Bereiche beider Branchen an der Basis gelenkig angebracht und drehen sich um den Befestigungspunkt. Auch bei dieser Konstruktion wird der Clip appliziert, indem der distale Rand des Schafts an den Gleitflächen, welche an den Außenseiten der Branchen vorgesehen sind, abgleitet und so die Branchen nach innen drängt. Das Problem dieser Art von Instrumentenköpfen ist, dass sie eine immer gleiche Schließgeometrie aufweisen, genauer gesagt, dass sich immer zuerst die distalen Enden der Branchen berühren bzw. aneinander vorbei gleiten und sich der Kontakt bzw. das aneinander vorbei Gleiten der weiter proximal liegenden Bereiche der Branchen dem anschließt. Bei Clipapplikatoren bedeutet dies, dass der Clip immer vom distalen Ende her geschlossen wird. Für andere chirurgische Instrumente wie beispielsweise eine endoskopische Schere ist dieser Aufbau eines Instrumentenkopfes aus diesem Grund nicht brauchbar. Ein weiteres Problem dieser Art von Instrumentenkopf ist, dass das Öffnen des Instrumentenkopfes alleine durch die Elastizität der Branchen realisiert wird. Die Öffnungsbewegung des Instrumentenkopfes erfolgt ohne Führung. Sollte ein Gewebestück oder ein anderes Teil zwischen den vorderen Rand des Schaftes und eine Branche des Instrumentenkopfes gelangen, könnte dies den Öffnungsvorgang des Instrumentenkopfes behindern. Dann müsste das Instrument erst aus dem Hohlraum im Inneren des Patienten entnommen werden, um von dem Gewebestück befreit zu werden, um anschließend wieder in den Patienten eingeführt zu werden. Dies führt zu Verzögerungen und Störungen im Operationsablauf.

Die Aufgabe die in einer parallelen Patentanmeldung gelöst ist, ist einen Instrumentenkopf für ein chirurgisches Rohrschaft-Instrument bereit zu stellen, bei dem zum einen die Schließgeometrie des Instrumentenkopfes beliebig einstellbar ist und bei dem zum anderen ein geführtes Schließen und Öffnen der Maulteilbranchen erfolgt.

Diese Aufgabe ist an einem Gerät mit einem Maulteil für ein chirurgisches Rohrschaft-Instrument, mit einem Haltebauteil, einer ersten Branche mit einem ersten Wirkbereich, und einer zweiten Branche mit einem zweiten Wirkbereich, dadurch gelöst, dass die erste Branche und/oder die zweite Branche über je ein Kulissenelement verfügt und die Branchen durch das Haltebauteil in axialer Richtung gehalten sind, wobei ein Nockenträgerbauteil / Schieber vorgesehen ist, welches / welcher relativ zu dem Haltebauteil in axialer Richtung verschiebbar ist und mindestens zwei Nocken trägt, wobei jedes Kulissenelement daran angepasst ist, bei einer relativen axialen Verschiebung zwischen dem Haltebauteil und dem Nockenträgerbauteil mit mindestens zwei Nocken, die an dem Nockenträgerbauteil / dem Schieber vorgesehen sind, in Kontakt zu stehen und an diesen abzugleiten, um so ein Öffnen bzw. Schließen der Maulteilbranchen zu bewirken.

Die hier vorliegende Erfindung betrifft gerade auch ein medizinisches Schaftinstrument mit einem solchen Instrumentenkopf.

Dabei ist es von Vorteil, wenn der Instrumentenkopf dadurch weitergebildet wird, dass das Haltebauteil einstückig mit einem Schaftbauteil des Schafts ausgebildet ist oder an diesem befestigt ist und dass das Nockenträgerbauteil ein Schieber ist, welcher relativ zu dem Schaft axial bewegbar ist. Die eine Maulteilbranche / erste Branche und die andere Maulteilbranche / zweite Branche sind vorzugsweise elastisch gekoppelt. Vorteilhaft können an wenigstens einem Kulissenelement mindestens zwei Kulissenbahnen ausgebildet sein. Die erste Branche und/oder die zweite Branche verfügt bspw. über wenigstens einen Vorsprung, der in einen Bereich des Haltebauteils eingreift und so eine axiale Bewegung der Branche gegenüber dem Haltebauteil begrenzt und bevorzugt verhindert.

Der wenigstens eine Vorsprung kann an einem biegeelastischen Fortsatz des zugehörigen Kulissenelements der Branche vorgesehen sein und der biegeelastische Fortsatz kann den Vorsprung zu dem Haltebauteil hin drängen und so einen Eingriff des wenigstens einen Vorsprungs in das Haltebauteil sichern, wobei die Biegeelastizität des Fortsatzes derart eingestellt ist, dass die Bewegbarkeit und die Bewegungen des Kulissenelements und des Wirkbereichs durch den Fortsatz im Wesentlichen nicht beeinflusst sind. Mindestens ein Kulissenelement kann im Wesentlichen flächig ausgebildet sein; das Nockenträgerbauteil kann im Wesentlichen flächig ausgebildet sein und das mindestens eine Kulissenelement kann im Wesentlichen nur an einer flachen Seite des Nockenträgerbauteils anliegen, das als sandwichartiger Aufbau ausgebildet ist, wobei bevorzugt je ein Kulissenelement auf beiden Seiten des Nockenträgerbauteils angeordnet ist.

Das Nockenträgerbauteil und mindestens ein Kulissenelement können mindestens einen Bereich ausbilden, indem eine Kulissenbahn und die zugehörige Nocke des Nockenträgerbauteils eine Hinterschneidung ausbilden, so dass ein Abheben des Kulissenelements von dem Nockenträgerbauteil verhindert ist, wobei bevorzugt mindestens ein Bereich einer Hinterschneidung über den gesamten Bewegungsbereich des Kulissenelements zu dem Nockenträgerbauteil von einer vollständig geöffneten Stellung zu einer vollständig geschlossenen Stellung des Instrumentenkopfes vorhanden ist. Ferner kann das chirurgische Instrument ein chirurgischer Clipapplikator sein und die Branchen des Instrumentenkopfes daran angepasst sein, einen chirurgischen Clip zu halten und durch ein Schließen des Instrumentenkopfes zu applizieren, wobei der chirurgische Clip vorzugsweise ein Doppelsteg-Clip ist, der weiter vorzugsweise aus zwei Cliphälften besteht, die jeweils nur an ihren beiden distalen Enden miteinander (fest) verbunden sind. Die Wirkbereiche der beiden Branchen des Instrumentenkopfes können daran angepasst sein. In der vollständig geöffneten Stellung des Instrumentenkopfes durch einen Clip, der im Instrumentenkopf angeordnet ist, nach außen über die laterale Position der Wirkbereiche der Branchen hinaus verschoben zu werden, welche die Wirkbereiche in der vollständig geöffneten Stellung des Instrumentenkopfes einnehmen, wenn kein Clip im Instrumentenkopf angeordnet ist. An mindestens einem Kulissenelement können mindestens drei Kulissenbahnen ausgebildet sein, wobei zu jedem Zeitpunkt während des Öffnungs- und Schließvorgangs der Instrumentenkopf mindestens zwei Kulissenbahnen an jeweils einer Nocke anliegen, welche an dem Nockenträger vorgesehen sind.

Der Instrumentenkopf kann einen vorzugsweise austauschbar anbringbaren Clipsspeicher / ein Clipmagazin aufweisen, indem eine Vielzahl von Clips / Klammern vorgesehen sind, wobei der Clipsspeicher / das Clipmagazin zumindest teilweise in einer Ebene angeordnet ist, die parallel zu einem sandwichartigen Aufbau des mindestens einen Kulissenelements mit dem Nockenträgerbauteil ist, wobei die Clips zumindest teilweise in einem Clipsspeicher einem sandwichartigen Schichttaufbau vorbei zu den distalen Bereichen der Branchen zuführbar sind. Zumindest die Wirkbereiche der ersten Branche und der zweiten Branche weisen eine zu einer Mittelachse des Instrumentenkopfes symmetrische Bewegungskurve in einer speziellen Ausführungsform auf. Das chirurgische Instrument kann als Schere, als Nadelhalter, als Klemme oder ein ähnliches chirurgisches Instrument ausgebildet sein, bei dem zwei Branchen aufeinander zu und/oder aneinander vorbei bewegbar sind. Es sei angemerkt, dass die symmetrische Bewegungskurve nicht zwingend symmetrisch sein muss, sondern einstellbar sein kann. Es kann sogar sein, dass sich eine Branche zur Symmetrielinie hin bewegt und die andere weg, allerdings langsamer.

Der Instrumentenkopf kann auch für ein chirurgisches Rohrschaft-Instrument mit einer ersten Branche mit einem ersten Wirkbereich, mit einer zweiten Branche mit einem zweiten Wirkbereich ausgebildet sein und derart weitergebildet sein, dass die erste Branche und/oder die zweite Branche über je ein Kulissenelement verfügt und die Branchen in axialer Richtung gehalten sind, und ein Nockenträgerbauteil vorgesehen ist, welches relativ zu dem mindestens einen Kulissenelement in axialer Richtung verschiebbar ist, wobei das mindestens eine Kulissenelement und das Nockenträgerbauteil im Wesentlichen flächig ausgebildet sind und im Wesentlichen schichtartig übereinander angeordnet sind. Das Nockenträgerbauteil und mindestens ein Kulissenelement können mindestens einen Bereich aufweisen, indem sie eine Hinterschneidung ausbilden, so dass ein Abheben des Kulissenelements von dem Nockenträgerbauteil verhindert ist, wobei bevorzugt mindestens ein Bereich einer Hinterschneidung über den gesamten Bewegungsbereich des Kulissenelements zu dem Nockenträgerbauteil von einer vollständig geöffneten Stellung zu einer vollständig geschlossenen Stellung des Maulteils vorhanden ist.

Das Nockenträgerbauteil kann wenigstens eine Nocke tragen, die wenigstens über eine Teilaxialverschiebung des Nockenträgerbauteils zu dem Kulissenelement an einer Kulissenbahn anliegt, welche an dem Kulissenelement ausgebildet ist, wobei die Nocke auf der der Kulissenbahn zugewandten Seite ihres axialen Querschnitts im Wesentlichen eine Z-Form, eine S-Form oder eine Kombination daraus aufweist. Nicht unerwähnt bleiben soll, dass das Nockenträgerbauteil wenigstens eine Nocke tragen kann, die wenigstens über einen Teil der Axialverschiebung des Nockenträgerbauteils zu dem Kulissenelement an einer Kulissenbahn anliegt, welche an dem Kulissenelement ausgebildet ist, wobei die Kulissenbahn auf der der Nocke zugewandten Seite ihres axialen Querschnitts im Wesentlichen eine Z-Form, eine S-Form oder eine Kombination daraus aufweist. Selbst die Nocke und die Kulissenbahn können an den jeweils zugewandten Seiten ihres Querschnitts im Wesentlichen eine S-Form aufweisen und die Nocke und die Kulissenbahn auf diese Weise wie einen Bauch und eine Kehle ausbilden und der Bauch eines Bauteils jeweils in die Kehle des anderen Bauteils vorstehen.

Zwischen dem Bauch und der Kehle der Nocke und/oder der Kulissenbahn kann ein gerader Abschnitt ausgebildet sein, wobei dieser gerade Abschnitt vorzugsweise gegenüber der Öffnungs- / Schließrichtung des Instrumentenkopfes geneigt ist, weiter vorzugsweise mehr als 7°, insbesondere weniger als 20°. Die Krümmung des Bauchs der Kulissenbahn kann kleiner als die Krümmung der Kehle der Kulissenbahn sein und/oder die Krümmung des Bauchs der Nocke kann größer als die Krümmung der Kehle der Nocke sein. Die Krümmung des Bauchs der Kulissenbahn kann größer als die Krümmung der Kehle der Nocke sein und/oder die Krümmung des Bauchs der Nocke kann größer als die Krümmung der Kehle der Kulissenbahn sein. Die Richtung der Krafteinleitung von der Nocke in die Kulissenbahn gegenüber der Öffnung- / Schließrichtung des Instrumentenkopfes kann geneigt sein, vorzugsweise in einem Bereich von bis zu 20°. Ferner kann die Oberfläche der Nocke im Bereich des Kontaktpunkts mit der Kulissenbahn der Mittelachse des Nockenträgerbauteils zugewandt sein. Wenn sich mindestens ein Kulissenelement im Wesentlichen über die gesamte Breite des Nockenträgerbauteils erstreckt, so dass die jeweilige Nocke des Nockenträgerbauteils im Wesentlichen durch das entsprechende Kulissenelement bedeckt ist, so wird die Vorrichtung vorteilhaft weitergebildet.

Mindestens ein Kulissenelement kann wenigstens zwei Kulissenbahnen aufweisen, welche mit einer entsprechenden Anzahl von Nocken an dem Nockenträgerring über einen Teil der Axialverschiebung der beiden Bauteile zueinander von einer vollständig geöffneten Stellung zu einer vollständig geschlossenen Stellung des Instrumentenkopfes je eine Hinterschneidung ausbilden, wobei ein Nockenträgerbauteil und ein Kulissenelement durch die Hinterschneidungen ausschließlich zusammenbaubar sind, indem sie ineinander eindrehbar sind oder ineinander eingedreht sind.

Falls sich das Kulissenelement und das Nockenträgerbauteil nach einem Eindrehen und Zusammenbauen einer Montagestellung befinden, die sich außerhalb der relativen Axialverschiebung des Kulissenelementes und des Nockenträgerbauteils zueinander von einer vollständig geöffneten Stellung zu einer vollständig geschlossenen Stellung des Bauteils befindet, wird eine weiter optimierte Ausführungsform erreichbar. Die erste Branche und die zweite Branche können über je ein Kulissenelement verfügen und die Branchen in axialer Richtung gehalten sein, und ein Nockenträgerbauteil vorgesehen sein, welches relativ zu dem mindestens einen Kulissenelement in axialer Richtung verschiebbar ist, wobei die Kulissenelemente im Wesentlichen flächig ausgebildet sind und im Wesentlichen schichtartig übereinander angeordnet sind und das Nockenträgerbauteil im Wesentlichen einen hohlen Querschnitt aufweist, in dem die Kulissenelemente angeordnet sind. Das Kulissenbauteil und das Nockenträgerbauteil können in einer Montagestellung durch eine Axialbewegung relativ zueinander in eine vollständig geöffnete Stellung verbracht sein / verbringbar sein.

Vorteilhaft ist es in diesem Zusammenhang, ein Verfahren zum Zusammenbau einer Branche und eines Nockenträgerbauteils für einen Instrumentenkopf einzusetzen, das folgende Schritte einsetzt: Bereitstellen einer Branche und eines Nockenträgerbauteils in einem Zustand, in dem ein Kulissenelement der Branche der zugehörigen Seite des Nockenträgerbauteils zugewandt ist, wobei die Branche und das Nockenträgerbauteil in zwei parallelen Ebenen angeordnet sind, die voneinander beabstandet sind, und die Längsachsen der beiden Bauteile einen bestimmten Winkel zwischen sich einnehmen, Heranführen der Branche und des Nockenträgerbauteils aneinander, bis diese in Kontakt gelangen, und Drehen der Branche und des Nockenträgerbauteils relativ zueinander in der durch die ihre Kontaktfläche bestimmte Ebene in der Weise, dass sich der Winkel zwischen ihren Längsachsen verringert, bis mindestens je eine Nocke des Nockenträgerbauteils beiderseits des Kreuzungspunkts der beiden Längsachsen in Kontakt mit einer Kulissenbahn gelangt.

Ein Schaftbauteil und ein Befestigungsstab lassen sich hinzufügen, mit folgenden Schritten: Bereitstellen einer Branche und eines Nockenträgerbauteils in einem Zustand, in dem ein Kulissenelement der Branche der zugehörigen Seite des Nockenträgerbauteils zugewandt ist, wobei die Branche und das Nockenträgerbauteil in zwei parallelen Ebenen angeordnet sind, die voneinander beabstandet sind, und die Längsachsen der beiden Bauteile einen bestimmten Winkel zwischen sich einnehmen, Heranführen der Branche und das Nockenträgerbauteils aneinander, bis diese in Kontakt gelangen, Drehen der Branche und des Nockenträgerbauteils relativ zueinander in der durch die ihre Kontaktfläche bestimmten Ebene in der Weise, dass sich der Winkel zwischen ihren Längsachsen verringert, bis mindestens je eine Nocke des Nockenträgerbauteils beiderseits des Kreuzungspunkts der beiden Längsachsen in Kontakt mit einer Kulissenbahn der Branche gelangt, Anbringen einer anderen Branche an der gegenüberliegenden Seite des Nockenträgerbauteils, wobei dieser Schritt auch zu Beginn des Verfahrens erfolgen kann, und Einschieben des proximalen Endes des Instrumentenkopfes in ein distales Ende des Schaftbauteils, wobei die Branche und/oder die andere Branche sowie das Nockenträgerbauteil zu unterschiedlichen Zeitpunkten mit einem von dem Schaftbauteil und dem Betätigungsstab in Eingriff gelangen, so dass dadurch eine axiale Verschiebung zwischen dem Nockenträgerbauteil sowie der Branche und/oder der anderen Branche in eine Stellung erfolgt, die der vollständig geöffneten Stellung des Instrumentenkopfes entspricht.

Die medizinischen Schaftinstrumente der eingangs genannten Art sind aber noch bzgl. der Führung der Klammern oder Clips zu optimieren. Die Klammern sollen präziser förderbar sein und ein Verklemmen verhindert werden.

Es ist die Aufgabe der vorliegenden Erfindung, ein möglichst ruckfreies Betätigen der Klammern zu ermöglichen, insbesondere ein reib- und/oder ruckarmes Bewegen der Klammern zu ermöglichen und Kraftsprünge zu vermeiden. Ein ruckfreies Fördern soll erreicht werden.

Diese Aufgabe wird erfindungsgemäß dadurch gelöst, dass der Relativabstand der einzelnen Klammer-Mitnahmeelemente, in Axialrichtung gesehen, unterschiedlich zu den jeweiligen Lagerpositionsabständen der Klammern ist, derart, dass die Mitnahme der gebunkerten Klammern im Rahmen eines einzigen Förderhubs getaktet / zeitlich versetzt erfolgt. Die Kraftentwicklung im Gerät entwickelt sich dann im Betrieb geschickt und vermeidet Stöße und Rücke.

Die vorliegende Erfindung ist durch die Merkmale des unabhängigen Anspruchs definiert. Vorteilhafte Ausführungsformen sind in den Unteransprüchen beansprucht und werden nachfolgend näher erläutert.

Vorteilhaft für den Kraftverlauf ist es dabei auch, wenn jene die klammerfördernden und führenden Einrichtungen, wie die Förder- und Mitnahmeschiene sowie eine Rückhalteschiene / ein Rückhalteblech, und die Klammern so aufeinander abgestimmt sind, dass die Klammern in anderen Abständen zwischen zwei Förderhüben im Schaftinstrument abgelegt werden, als durch die fördernden Einrichtungen (alleine), wie die Klammer-Mitnahmeelemente oder ähnliche Vorschubelemente, vorgegeben ist.

Von Vorteil für die Präzision ist es auch, wenn die Klammer-Mitnahmeelemente, die Lagerpositionen, die von Rückhaltelaschen einer Rückhalteschiene / Auflagerschiene vorbestimmt sind, die Klammern und die die Klammern bei ihrer Bewegung nach distal bewegende Förder- und Mitnahmeschiene so aufeinander abgestimmt sind, dass als erstes eine proximale Klammer und zeitlich nachfolgend während des Förderhubes die distal vorgelagerten Klammern (einer nach der anderen) bewegt werden oder als erstes eine distale Klammer und zeitlich nachfolgend die proximal nachgelagerten Klammern (eine nach der anderen) bewegt werden.

7

Die Abstände zwischen den Klammern-Mitnahmeelementen sind gemäß der Erfindung (in Axialrichtung gesehen) unterschiedlich und können bspw. entweder in Richtung eines distalen Endes des Instrumentenkopfs gleichmäßig oder sprunghaft zunehmen oder gleichmäßig oder sprunghaft abnehmen.

Gemäß der Erfindung sind die Abstände zwischen den Rückhaltelaschen (in Axialrichtung gesehen) unterschiedlich. Und wenn diese Abstände zwischen den Rückhaltelaschen vorzugsweise entweder in Richtung eines distalen Endes des Instrumentenkopfes gleichmäßig oder sprunghaft zunehmen oder gleichmäßig oder sprunghaft abnehmen, wird eine weitere vorteilhafte Ausführungsform erreichbar.

Dabei ist es zweckmäßig, wenn die Abstände zwischen den einzelnen Klammer-Mitnahmeelementen den Abständen zwischen den einzelnen Rückhaltelaschen (exakt) entsprechen oder gemäß der Erfindung die relative Veränderung der Abstände zwischen den Klammer-Mitnahmeelementen einerseits und den Rückhaltelaschen andererseits kleiner oder größer ist.

Ein vorteilhaftes Ausführungsbeispiel ist auch dadurch gekennzeichnet, dass die Rückhaltelaschen beim Überfahrenwerden von den Klammern während des von den Klammern zurückgelegten Weg zum distalen Ende des Instrumentenkopfes aus dem Bewegungspfad der Klammern ausreichend biegbar oder verschwenkbar an der Rückhalteschiene angebunden sind.

Damit nicht zu viele Reibkräfte auftreten, ist es von Vorteil, wenn die Rückhaltelasche nach Art von Schmetterlingslaschen um eine quer zur Längsrichtung der Rückhalteschiene ausgerichtete Biege- oder Schwenkachse biegbar oder schwenkbar, vorzugsweise als integrale Bestandteile der Rückhalteschiene angebunden sind. Das Konzept der Biegbarkeit und /oder Schwenkbarkeit, wie es bei den Rückhaltelaschen realisiert wird, ist auch auf die Klammermitnahmeelemente übertragbar.

Vorteilhaft ist es auch, wenn zwei Flügel einer Schmetterlingslasche eine Rückhaltelasche ausbilden, wobei jeder Flügel um eine Biege- oder Schwenkachse biegbar oder schwenkbar ist, die sich distal oder proximal der Rückhaltelasche kreuzen. Ein besonders geschicktes Ausweichverhalten der Rückhaltelaschen kann dann bedingt werden.

Wenn die Rückhaltelaschen geometrisch und stofflich so ausgestaltet sind, dass sie sich beim Überfahren werden durch die Klammern (näherungsweise) flach legen, d.h. in Axialrichtung ausgerichtet verlaufen, und vorzugsweise nicht durch die Rückhalteschiene hindurch von den Klammern weg ragen, baut das Schaftinstrument besonders schmal. Zusatzfunktionalitäten lassen sich integrieren, wenn die quer zur Axialrichtung gemessene Höhe zumindest einiger der Rückhaltelaschen so groß ist, dass die eine durch die Klammern hindurchgeführte Zunge / Vorschubzunge zum Ausstoßen der distalsten Klammer führt und/oder ein Ausknicken der Zunge verhindert / eindämmt.

Besonders bewährt hat es sich, dass zwischen zwei Klammer-Mitnahmeelementen oder zwei Rückhaltelaschen in Richtung distal (und /) oder proximal die Abstände abnehmen oder zunehmen. Besonders vorteilhaft ist es, wenn die durchschnittliche Abstandsänderung der die Klammern kontaktierenden Formschlusselementen zum durchschnittlichen Abstand etwa 0,1% bis 4% beträgt. Vorzugsweise jedoch 0,8% bis 1,5% beträgt, wobei die Formschlusselemente ein Teil der Förder- und Mitnahmeschiene oder der Rückhalteschiene sind.

Wenn die Rückhalteschiene als Rückhalteblech aus einem metallischen Werkstoff ausgebildet ist, wird eine besonders gute Stabilität erreicht. Natürlich kann alternativ auch Kunststoff eingesetzt werden.

In diesem Zusammenhang kann die Erfindung auch noch anders weitergebildet werden. So betrifft die Erfindung auch ein medizinisches Schaftinstrument, vorzugsweise der Minimalinvasiv-Bauart, mit einem Instrumentenkopf zum Applizieren von Klammern, Clips, Klipsen oder (Feder-)Klemmen, wobei der Instrumentenkopf über einen Instrumentenschaft mit einem Instrumentengriff zur Betätigung des Instrumentenkopfes verbindbar ist, und mit einem Clipmagazin, in welchem eine Rückhalteschiene / Auflageschiene zur Auflagerung / zum lagernden Ablegen einer Anzahl von Klammern nach dem Einlagerungsprinzip / Einzellagerungsprinzip in einem vorbestimmten Lagerpositionsabstand zueinander fixiert ist, wobei die Klammern so eingelagert sind, dass sie sämtliche mittels einer hin- und herbewegbaren Förder- und Mitnahmeschiene während eines einzigen Förderhubs um jeweils (genau) eine Lagerposition vorrückbar sind / vorgerückt werden und von denen die dem Instrumentenkopf nächstgelegene Klammer mittels einer Zunge / Vorschubzunge in den Instrumentenkopf zum Ausstoß und zur Verbiegung förderbar ist / gefördert ist. Diese Weiterentwicklung ist dadurch gekennzeichnet, dass insbesondere im Clipmagazinbereich die Rückhalteschiene, die Förder- und Mitnahmeschiene und die Zunge in einer Schichtbauweise (zueinander / übereinander) angeordnet sind.

Es ist von Vorteil für eine kompakte Bauform, wenn die Rückhalteschiene, die Förder- und Mitnahmeschiene und die Zunge in einer quer / orthogonal zur durch die Längsachse des Schaftinstruments festgelegten Richtung übereinander geschichtet angeordnet sind und sich vorzugsweise im Wesentlichen in Richtung der Längsachse, die die Axialrichtung vorgibt, erstrecken.

Es ist dabei von Vorteil für ein gutes Funktionieren, wenn die Zunge zwischen der Rückhalteschiene und der Förder- und Mitnahmeschiene angeordnet ist.

Wenn am distalen Ende des Instrumentenkopfes ein Maulteil ausgebildet ist, in das die distale Klammer von der Zunge zur Interaktion mit einem zu behandelnden Element, Gefäß oder Organ verbringbar ist / verbracht ist, wird ein effizienter medizinischer Einsatz vor Ort bei einer Ligatur ermöglicht.

Die Erfindung kann auch weitergebildet werden. So betrifft die Erfindung auch ein medizinisches Schaftinstrument, vorzugsweise der Minimalinvasiv-Bauart, mit einem wenigstens ein Maulteil aufweisenden Instrumentenkopf zum Applizieren von Klammern, Clips, Klipsen oder Federklemmen, wobei der Instrumentenkopf über einen ein Außenrohr aufweisenden Instrumentenschaft mit einem Instrumentengriff (u.a.) zur Betätigung / Aktuierung des Maulteils verbunden ist, und mit einem Clipmagazin, in welchem eine Rückhalteschiene / Auflageschiene zur Auflagerung einer Anzahl / Vielzahl an Klammern nach dem Einlagerungsprinzip / Einzellagerungsprinzip mit einem vorbestimmten Lagerpositionsabstand zueinander fixiert ist, wobei sämtliche Klammern mittels einer hin- und herbewegbaren Förder- und Mitnahmeschiene im Rahmen eines einzigen Förderhubs um jeweils (genau) eine Lagerposition (in Richtung des distalen Endes) vorrückbar sind, und von denen die den Instrumentenkopf nächstgelegene Klammer mittels einer Zunge / Vorschubzunge in das Maulteil beförderbar ist. Die Weiterbildung ist dadurch gekennzeichnet, dass die Förder- und Mitnahmeschiene über eine Kopplungseinrichtung so an der Zunge oder einem Vorschubaufteiler angebunden ist, dass im Fall einer ausgelösten Vorschubbewegung für einen Förderhub der Zunge bei Erreichen oder Überschreiten eines bestimmten / vorbestimmten Vorschubweges die Förder- und Mitnahmeschiene mitgenommen wird.

Die Fertigung lässt sich vereinfachen, wenn die Kopplungseinrichtung nach Art eines Vorschubaufteilers als von der Zunge und der Förder- und Mitnahmeschiene separates Bauteil ausgebildet ist oder als mit der Zunge oder der Förder- und Mitnahmeschiene einstückiges (integrales und/oder einstoffliches) Bauteil ausgebildet ist.

Vorteilhaft ist es ferner, wenn die Kopplungseinrichtung eine Nocken-Langloch-Kombination zur verzögerten Übertragung von Kräften und Bewegungen von einer Vorschubstange auf die Förder- und Mitnahmeschiene einsetzt, wobei die Zunge direkt / fest mit einem Antriebsstrang verbunden ist.

Es ist zweckmäßig, wenn ein zungenseitiger Nocken in ein förder- und mitnahmeschienenseitiges Langloch zum Eingeben eines Formschlusses greift.

Die Erfindung ist auch Gegenstand einer Weiterentwicklung. Sie betrifft auch ein medizinisches Schaftinstrument, vorzugsweise der Minimalinvasivbauart, mit einem wenigstens ein Maulteil aufweisenden Instrumentenkopf zum Applizieren von Klammern, Clips, Klipsen oder (Feder-)Klemmen, der über einen ein Außenrohr aufweisenden Instrumentenschaft mit einem Instrumentengriff zur Betätigung / Aktuierung des Instrumentenkopfes verbindbar ist und einem Clipmagazin, in welchem eine Rückhalteschiene / Auflagerschiene zur Auflagerung von Klammern nach dem Einlagerungsprinzip / Einzellagerungsprinzip mit einem vorbestimmten Lagerpositionsabstand zueinander fixiert ist, wobei die Klammern sämtlich mittels einer hin- und herbewegbaren Förder- und Mitnahmeschiene im Rahmen eines einzigen Förderhubs um jeweils eine (einzige) Lagerposition vorrückbar sind, wofür an der Rückhalteschiene und an der Förder- und Mitnahmeschiene voneinander beabstandete Rückhaltelaschen einerseits und Klammer-Mitnahmeelemente andererseits vorgesehen sind, welche beim Übergleiten der Klammern in die jeweils andere Richtung federelastisch aus der Klammergleitbahn wegschwenkend ausgestaltet sind. Diese Weiterentwicklung ist dadurch gekennzeichnet, dass alle oder eine Anzahl der Rückhaltelaschen und/oder Klammer-Mitnahmeelemente in einer Schmetterlingsform ausgebildet sind, die mit zwei in Förderrichtung / Förderhubrichtung / Vorschubrichtung zueinander abgekippten Anschlagsflügeln ausgebildet sind, und/oder eine Anzahl der Rückhaltelaschen und/oder Klammer-Mitnahmeelemente in einer Stützkeilform ausgebildet sind, mit einer quer zur Förderrichtung verschwenkbaren Kuppe, an deren distalen freien Kante.

Vorzugsweise ist dabei eine im Wesentlichen senkrecht oder zumindest schräg zur Förderrichtung verlaufende Anschlagsplatte oder Anschlagsfläche an den Rückhaltelaschen und/oder Klammer-Mitnahmeelementen ausgebildet, etwa im Bereich der Kuppe.

Zweckmäßig ist es, wenn die Zunge durch die Förder- und Mitnahmeschiene und/oder die Rückhalteschiene abgestützt ist.

Vorteilhaft ist es auch, wenn von der Rückhalteschiene in Richtung der Zunge diese unterstützende Federlaschen abstehen, vorzugsweise zwei nebeneinander in der Gegend des distalen Endes der Zunge, wobei überwiegend beide in Richtung proximal weisen oder eine in Richtung proximal weist, während die andere in Richtung distal weist.

Die Erfindung betrifft auch eine Weitereentwicklung, nämlich ein medizinisches Schaftinstrument, das auch als Rohrschaft-Instrument bezeichnet werden kann, vorzugsweise der Minimalinvasivbauart, mit einem Instrumentenkopf zum Applizieren von Klammern, etwa Ligaturklammern, Clips, Klipsen oder Klemmen, wobei diese zu applizierenden Bauteile zur plastischen Verformung vorbereitet sind, wobei des weiteren der Instrumentenkopf über einen ein Außenrohr / Mantelrohr aufweisenden Instrumentenschaft mit einem Instrumentengriff zur Betätigung / Aktuierung des Instrumentenkopfs verbindbar ist, und mit einem ein Gehäuse aufweisenden Clip-Magazin, in welchem eine Anzahl / Vielzahl der Klammern nach dem Einlagerungsprinzip / Einzellagerungsprinzip bunkerbar bzw. lagerbar / gelagert sind.

Die Weiterentwicklung besteht darin, dass das Clipmagazin in dem Instrumentenschaft integriert ist, indem das Gehäuse des Clipmagazins durch das Außenrohr des Instrumentenschafts selbst gebildet ist, an dem sich die Klammern gleitfähig wenigstens abschnittsweise abstützen oder an dem die Klammern gleitfähig wenigstens abschnittsweise abstützbar sind.

Diese Weiterbildung ist geschickt durch Ergänzungen noch weiter verbesserbar.

So ist es von Vorteil, wenn am Außenrohr zumindest zwei in Umfangsrichtung voneinander beabstandete Berührungsflächen / Anlageflächen zur Gleitbewegung ermöglichenden Anlage einer Klammer oder mehrerer Klammern ausgebildet sind. Über den ganzen Weg während eines Führungshubes oder nur einem Teilweg kann somit eine relativ reibarme Gleitfläche für die Klammern auf einer ihrer Außenseiten zur Verfügung gestellt werden.

Es ist dabei von Vorteil, wenn zwei, drei oder vier in Umfangsrichtung voneinander beabstandete Berührungsflächen bspw. mit einer der Klammern oder allen Klammern ausgebildet sind. Durch die Vielzahl an Berührungsflächen wird eine hohe Verkippsicherheit erreicht.

Zweckmäßig ist es ferner, wenn jeweils zwei Berührungsflächen zum Kontaktieren eines Schenkels der Klammer, vorzugsweise an unterschiedlichen Stellen, vorbereitet sind. Das Außenrohr ist also speziell und explizit darauf vorbereitet, in direkte Anlage mit Klammerabschnitten zu gelangen, um ein Vorwärtsgleiten in präziser Art zu ermöglichen. Wenn einerseits die Berührungsflächen auf der Innenseite des Außenrohres oder andererseits an etwa in Längsrichtung ausgerichteten, rohrinnenseitigen Schlitzen und/oder Nuten in dem Außenrohr ausgeformt sind, kann einerseits ein Durchragen oder Durchdringen von Klammern verhindert werden bzw. andererseits das Außenrohr vom Durchmesser her kleiner gewählt werden, so dass eine besonders kompakte Bauform bewirkt ist.

Eine vorteilhafte Ausführungsform kann sich im zweiten der beiden Fälle dadurch gestaltet sein, dass sich die Schlitze komplett durch die Wandung des Außenrohres hindurch erstrecken bzw. die Nuten, etwa radial außenseitig, einseitig verschlossen sind, und einerseits entweder nur Prägungsabschnitte, Sicken, Bördelungen, Schlitze oder Nuten die Berührungsflächen ausbilden oder andererseits die Schlitze und die Nuten die Berührungsflächen ausbilden. So können bestimmte Schenkel der Klammer / des Clips durch das Außenrohr ragen und andere ausschließlich innerhalb des Außenrohres gleitend angeordnet sein, was einen bedarfsgerechten Einsatz des Instruments ermöglicht.

Wenn das Außenrohr als ein versteifendes Blechrohr, etwa aus Edelstahl, ausgebildet ist oder als Kunststoffrohr / Glasrohr / Keramikrohr (runder oder eckiger Querschnitt) ausgebildet ist, lassen sich für den medizinischen Einsatz prädestinierte Materialien verwenden.

Das Außenrohr kann einen kreisringförmigen Querschnitt, einen eckigen Querschnitt oder einen ovalen Querschnitt aufweisen. Bedarfsgerechte und einsatzoptimierte Ausgestaltungen eines medizinischen Schaftinstrumentes lassen sich dann besser und vielfältig gestalten.

So ist es von Vorteil, wenn der Innendurchmesser des Außenrohres und/oder die Position der Berührungsflächen so auf die zu applizierenden Klammern abgestimmt ist / sind, dass die Klammer / Klammern bei der Nutzung bzw. im Inneren des Instruments vor Verlassen des Instruments zumindest kurzzeitig vorgespannt ist / werden.

Es ist von Vorteil, wenn eine Klammer oder mehrere Klammen eingesetzt sind, die mit zwei ihrer Klammerstegen / Stegen an den Berührungsflächen anliegen / anliegt. Die Klammer kann dabei als Doppelsteg-Clip oder alternativ als Einfachstegclip ausgebildet sein.

Die Erfindung betrifft auch eine Weiterentwicklung, nämlich ein medizinisches Schaftinstrument, vorzugsweise der Minimalinvasiv-Bauart, mit einem wenigstens ein Maulteil aufweisenden Instrumentenkopf zum Applizieren von Klammern, Clips, Klipsen oder (Feder)Klemmen, also plastisch verformbaren Abschnürelementen, mittels zweier scherenartig bewegbaren Maulteil-Branchen, wobei der Instrumentenkopf über einen ein Außenrohr aufweisenden Instrumentenschaft mit einem Instrumentengriff u.a. zur Betätigung / Aktuierung des Maulteils verbindbar ist und einem Clipmagazin, in welchem eine Rückhalteschiene / Auflagerschiene zur Auflagerung einer Anzahl von Klammern nach dem Einlagerungsprinzip / Einzellagerungsprinzip mit einem vorbestimmten Lagerpositionsabstand zueinander fixiert ist, wobei sämtliche Klammern mittels einer hin- und herbewegbaren Förder- und Mitnahmeschiene, etwa nach Art eines Vorschubbleches, im Rahmen eines einzigen Förderhubes um jeweils eine Lagerposition in Richtung des distalen Endes des Instrumentenschafts vorrückbar sind und von denen die dem Instrumentenkopf am nächsten liegende Klammer mittels einer Zunge / Vorschubzunge in das Maulteil zwischen die Maulteil-Branchen förderbar ist. Die Weiterentwicklung ist dadurch gekennzeichnet, dass die Rückhalteschiene eine sich in Richtung distal erstreckende Auflageplatte oder Brücke, etwa nach Art eines platten- oder stegförmigen Verlängerungsabschnitts aufweist, welche einen Maulteilbereich im Bereich der einen Maulteil-Branche auf der der anderen Maulteil-Branche zugewandten Seite so abdeckt, dass ein Abkippen einer vorgeschobenen oder vorschiebbaren Klammer auf die eine Maulteil-Branche verhindert ist / wird.

Ein Gefäß kann auch einfach abgedrückt werden, wenn die eine Maulteil-Branche eine schwerkraftbestimmt untere Maulteil-Branche ist.

Wenn eine der Maulteil-Branchen oder beide Maulteil-Branchen zur vorzugsweise formschlüssigen Aufnahme eines Abschnitts der Klammer vorbereitet ist / sind, so wird ein sicheres Applizieren möglich.

Die Klammer wird besonders sicher beim Verbiegen gehalten, wenn eine der Maulteil-Branchen oder jede der Maulteil-Branchen als Schale ausgebildet ist / sind, welche in Richtung der anderen Maulteil-Branche offen ist / sind.

Ferner ist es zum sicheren Leiten der Klammer von Vorteil, wenn die Auflageplatte oder die Brücke als Führungs- oder Leitorgan in Längsrichtung der Rückhalteschiene gesehen seitlich, etwa einseitig, absteht.

Die Erfindung betrifft auch eine Weiterentwicklung, nämlich ein medizinisches Schaftinstrument, vorzugsweise der Minimalinvasiv-Bauart, mit einem wenigstens ein Maulteil aufweisenden Instrumentenkopf zum Applizieren von Klammern, Clips, Klipsen oder (Feder-)Klemmen mittels zweier scherenartig bewegbarer Maulteil-Branchen, wobei der Instrumentenkopf über einen ein Außenrohr aufweisenden Instrumentenschaft mit einem Instrumentengriff u.a. zur Betätigung / Aktuierung des Maulteils verbindbar ist und einem Clipmagazin, in welchem eine Rückhalteschiene / Auflagerschiene zur Auflagerung einer Anzahl von Klammern nach dem Einlagerungsprinzip / Einzellagerungsprinzip mit einem vorbestimmten Lagerpositionsabstand zueinander fixiert ist, wobei sämtliche Klammern mittels einer hin- und herbewegbaren Förder- und Mitnahmeschiene, etwa nach Art eines Vorschubbleches, im Rahmen eines einzigen Förderhubs um jeweils eine Lagerposition in Richtung des distalen Endes des Instrumentenschafts vorrückbar sind und von denen die dem Instrumentenkopf am nächsten liegende Klammer mittels einer Zunge / Vorschubzunge in das Maulteil zwischen die Maulteil-Branchen förderbar ist. Die Weiterentwicklung ist dadurch gekennzeichnet, dass die Rückhalteschiene in ihrem distalen Endbereich einen kickerartigen / sprungschanzenartigen Abweiser zum Endbeschleunigen und/oder Umleiten der Klammer beim Verlassen der Rückhalteschiene und Eintauchen in einen Zwischenbereich zwischen die beiden Maulteil-Branchen aufweist.

Es ist von Vorteil, wenn der Abweiser sich nach Art einer Erhöhung in Richtung der Zunge und/oder der Förder- und Mitnahmeschiene erstreckt.

Zweckmäßig ist es auch, wenn der Abweiser als spanlos ausgeformte (Leit-)Sicke ausgebildet ist.

Ein vorteilhaftes Ausführungsbeispiel ist auch dadurch gekennzeichnet, dass der Abweiser in Richtung proximal angepfeilt ausgestaltet ist.

Es kann auch zwischen dem Abweiser und der Anlageplatte / Brücke ein Montageloch zum Angreifen eines Montagewerkzeugs vorgesehen sein. Ein solches zum Einhängen vorbereitetes Montagewerkzeug greift dann durch den Instrumentenschaft, insbesondere durch das Außenrohr hindurch, wodurch die Montage erleichtert wird.

Für die Montage ist es auch von Vorteil, wenn das Montageloch als Durchgangsloch mit rundem, ovalem oder eckigem Grundriss / Querschnitt ausgeformt ist.

Wenn der Abweiser, die Anlageplatte oder die Brücke, und die Klammer / die Klammern so aufeinander abgestimmt sind, dass ein proximaler Klammerabschnitt beim Übergleiten des Abweisers zwangsangehoben wird und distale Klammersteg-Spitzen zwangsabgesenkt werden, um ein blockierfreies Eingleiten in das Maulteil und vorzugweise die jeweilige Maulteil-Branchen zu begünstigen, so wird ein ruckelfreies Arbeiten mit dem medizinischen Schaftinstrument erleichtert.

Die Erfindung ist auch weiter entwickelbar, nämlich als medizinisches Schaftinstrument, vorzugsweise der Minimalinvasiv-Bauart, mit einem wenigstens ein Maulteil aufweisenden Instrumentenkopf zum Applizieren von Klammern, Clips, Klipsen, (Feder-) Klemmen, die durch plastische Verformung abklemmend auf Organe und Gefäße einwirken können, wobei der Instrumentenkopf über einen ein Außenrohr aufweisenden Instrumentenschaft mit einem Instrumentengriff u.a. zum Betätigen des Maulteils verbindbar ist, und mit einem Clipmagazin, in welchem eine Rückhalteschiene / Auflagerschiene zur Auflagerung einer Anzahl von Klammern nach dem Einlagerungsprinzip / Einzellagerungsprinzip mit einem bestimmten Lagerpositionsabstand zueinander fixiert ist, wobei sämtliche Klammern mittels einer hin- und herbewegbaren Förder- und Mitnahmeschiene im Rahmen eines einzigen Förderhubes um jeweils eine Lagerposition vorrückbar sind und von denen die dem Instrumentenkopf nächstgelegene Klammer mittels einer Zunge / Vorschubzunge in das Maulteil förderbar ist. Diese Weiterentwicklung ist dadurch gekennzeichnet, dass die Zunge als Stanz-Biegeteil aus einem Blechmaterial gefertigt ist, wobei die Zunge eine distale Endplatte aufweist, in der eine das Eindringen eines Abschnitts der Klammer in / durch die Zunge ermöglichende Eintauchausnehmung vorhanden ist. Auf diese Weise wird ein schmälerer Aufbau eines medizinischen Schaftinstruments ermöglicht und ein gleichmäßiger Ausstoß der Klammer erreicht.

Es ist von Vorteil, wenn die Eintauchausnehmung als Durchgangsloch, Sackloch, Schlitz, Nut oder Kerbe ausgebildet ist.

Dabei kann die Eintauchausnehmung einen kreisrunden, ovalen, rechteckigen, quadratischen oder polygonalen Grundriss / Querschnitt aufweisen.

Von Vorteil ist es auf jeden Fall, wenn die Endplatte seitliche Schiebekanten oder Drucklaschen aufweist, mit denen zusammen sie einen offenen Kastenquerschnitt vorgibt.

Zweckmäßig ist es, wenn der offene Kastenquerschnitt auf einen Biege-, Präge- und/oder einen Bördelungsfertigungsschritt zurück geht, so dass die Schiebekanten oder Drucklaschen aus umgebördeltem Blech ausgeformt sind.

Ein vorteilhaftes Ausführungsbeispiel ist auch dadurch gekennzeichnet, dass der Kastenquerschnitt so auf wenigstens das proximale Ende der Klammer abgestimmt ist, dass ein maximal langes Führen der Klammer beim Eintauchen in das Maulteil beim Verlassen der distalsten Lagerposition gewährleistet ist.

Wenn in einem Übergangsbereich zwischen der Zunge zu einem Maulteil eine Auflageplatte / Brücke für die Klammer vorhanden ist, die ein blockierfreies Übergleiten des Übergangsbereichs durch die Klammer sicherstellt, so kann mit relativ geringen Kräften intraoperativ gearbeitet werden.

Es ist von Vorteil, wenn die Zunge einen zentralen nach distal weisenden Gewebeabstandshalter aufweist.

Wenn sich der Gewebeabstandshalter über die distale Erstreckung der Schiebekanten oder Drucklaschen hinaus in Richtung distal erstreckt, so wird eine weitergehende vorteilhafte Ausführungsform realisierbar.

Die Erfindung ist auch Gegenstand noch einer Weiterentwicklung, die auch ein medizinisches Schaftinstrument, vorzugsweise der Minimalinvasiv-Bauart betrifft, mit einem (maulteilaufweisenden) Instrumentenkopf zum Applizieren von Klammern, Clips, Klipsen, (Feder-)Klemmen, wobei der Instrumentenkopf über einen ein Außenrohr aufweisenden Instrumentenschaft mit einem Instrumentengriff, u.a. zum Betätigen wenigstens eines Maulteils verbindbar ist, und mit einem Clipmagazin, in welchem eine Rückhalteschiene / Auflagerschiene zur Auflagerung einer Anzahl von Klammern nach dem Einlagerungsprinzip / Einzellagerungsprinzip mit einem bestimmten Lagerpositionsabstand zueinander fixiert ist, wobei sämtliche Klammern mittels einer hin- und herbewegbaren Förder- und Mitnahmeschiene im Rahmen eines einzigen Förderhubs um jeweils eine Lagerposition vorrückbar sind und von denen die dem Instrumentenkopf nächstgelegene Klammer mittels einer Zunge / Vorschubzunge in das Maulteil förderbar ist. Die Weiterentwicklung ist dadurch gekennzeichnet, dass die Zunge als Stanz-Biege-Teil aus einem Blechmaterial gefertigt ist, wobei auf einer Seite der Zunge im distalen Endbereich zwei stegartige Schiebekanten oder Drucklaschen vorhanden sind.

Es ist dabei für die problemlose Betätigung von Vorteil, wenn die Schiebekanten oder Drucklaschen auf die Form der Klammer abgestimmt sind.

Zweckmäßig ist es dabei, wenn die Schiebekanten oder Drucklaschen am Rand der Zunge, in Längsrichtung verlaufend, die Einfachausnehmung flankieren. Dadurch wird eine bessere Ausknickverhinderung erreicht.

Letztlich wird ein Doppelsteg-Clip-Applikator zur Verfügung gestellt. Natürlich kann dieser Clipapplikator auch Einzelstegclips applizieren. Die Funktionsweise eines solchen medizinischen Schaftinstrumentes wird nachfolgend kurz erläutert.

Wenn der Handgriff, wie ein "Challenger-Handgriff" betätigt wird, wird ein äußerer Kolben des Griffs axial nach distal bewegt und beaufschlagt ein direkt anliegendes Schubrohr mit einem Hub und einer Kraft in Achse zum Schaft liegend. Ein Federanschlag, der mit dem Schubrohr fest verbunden ist, wird gegen eine Rückholfeder geschoben, welche sich am Schaftrohr abstützt, wobei die Federkraft zunimmt. Das Schubrohr leitet die Bewegung direkt zu einem Schieber eines Maulteilpakets, welcher mittels einer Spange mit dem Schubrohr fest verbunden ist, und setzt diesen in Bewegung. Das Maulteilpaket kann auch als Instrumentenkopf bezeichnet werden. Der Schieber, befindlich zwischen zwei Maulteilschäften, die auch als Maulteil-Branchen bezeichnet werden können, wandelt die axiale Bewegung in eine Schließbewegung der Maulteile um. Nocken des Schiebers liegen an der Kulissenbahn der Maulteile an und verdrängen diese nach innen, woraufhin sich die Maulteile aufeinander zu bewegen und den dazwischen befindlichen Clip bzw. die dazwischen befindliche Klammer zusammen pressen.

Ist die Klammer verpresst, kann der Handgriff gelöst werden, was zur Folge hat, dass sich die Rückholfeder entspannt, das Schubrohr und somit den Schieber rückholt, und die Maulteile durch die im Eingriff befindlichen Nocken in die Aufwärtsstellung gezwungen werden.

Nachdem der Handgriff komplett entlastet ist, wird automatisch der Vorschub durch den pneumatischen Mechanismus des "Challenger-Griffs" ausgelöst.

Dabei wird das Ventil einer Gaskartusche geöffnet und ein innenliegender Vorschubkolben des Griffs beaufschlagt die Vorschubstange mit der axialen Bewegung und Vorschubkraft. Die Vorschubstange bewegt sich dann nach distal und wirkt gegen die Rückholfederkraft einer Druckfeder, die sich in der Vorschubbewegung spannt. Die Feder stützt sich hierbei auf der einen Seite an einem Absatz auf der Vorschubstange ab. Die andere Seite wird durch einen Federgegenhalter abgestützt, der durch entsprechende Laschen am Schaftrohr axial fixiert ist. Die Vorschubstange verläuft hierbei durch den Federgegenhalter.

Die in Bewegung versetzte Vorschubstange gibt die Bewegung direkt an den fest mit ihr verbundenen Vorschubverteiler weiter, welcher die Aufgabe hat, den Vorschubweg in zwei oder mehrere Beträge aufzuteilen.

Ist der Vorschubverteiler in Bewegung, leitet er die Bewegung direkt an die mit ihm fest verbundene Zunge, die den distalsten Clip aufsammelt und mitnimmt. Die Zunge ist hierbei durch alle Clips hindurchgeführt. Nach einem bestimmten Weg, welcher über ein Nockenfenster im Vorschubblech und einem Nocken am Vorschubverteiler definiert ist, bewegt sich das Vorschubblech ebenfalls nach distal. Der Hub des Vorschubbleches ist dadurch immer kleiner gleich dem der Zunge definiert.

Das in Bewegung versetzte Vorschubblech sammelt mit Hilfe der im Eingriff stehenden Vorschublaschen jeden einzelnen Clip aus der Einzelablage nacheinander (inkrementell) auf und setzt diese in Bewegung. Die Clips werden nun nach distal geschoben und überwinden die inkrementell angeordneten Rückhaltelaschen des Rückhalteblechs, wobei bei jedem Vorschubhub / Förderhub die Clips um jeweils eine Position nach distal befördert werden.

Gleichzeitig wird der distalste Clip durch die Zunge über die Clipführungsbahn in das Maul geschoben.

Ist der komplette Vorschubweg erreicht und alle Clips an ihrer vorbestimmten Position angekommen, läuft die Vorschubstange bzw. der Vorschubaufteiler auf einen Anschlag auf, bspw. läuft das Ende einer Nut in der Vorschubstange auf einen querliegenden Zylinderstift auf, was einen Anstieg der Kraft im Handgriff zur Folge hat. Erreicht diese Kraft ca. 40 N, wobei auch Werte von ca. 10 N, ca. 20 N, ca. 30 N, ca. 50 N, ca. 60 N und ca. 70 N (jeweils ± 5 N) denkbar sind, wird im Handgriff automatisch ein Ventil geöffnet, welches die Vorschubkraft auf null senkt und den Rückhub durch die Entspannung der gesamten Rückholfeder im Schaft einleitet.

Ist der Rückhub eingeleitet, wird die Vorschubstange nach proximal bewegt. Der fest mit der Vorschubstange verbundene Vorschubverteiler, sowie die fest verbundene Zunge, werden direkt nach proximal gezogen. Nachdem der Nocken des Vorschubverteilers den Weg im Nockenfeld des Vorschubbleches abgefahren ist, wird auch das Vorschubblech nach proximal bewegt.

Dabei werden die Vorschublaschen über die um eine Position verschobenen Clips / Klammern gezogen. Für den Fall, dass die Klammern während der Rückhubbewegung mitgezogen werden, dienen die dieser Bewegung entgegenstehenden Rückhaltelaschen des Rückhalteblechs um sie festzuhalten.

Die Zunge ist gleichzeitig im Rückhub durch den distalsten Clip hindurch zurückgezogen und kehrt in die Ausgangsposition zurück. Die Rückhubbewegung endet, wenn der Vorschubverteiler an die Gegenseite des Federanschlags der Rückholfeder anschlägt.

Nach diesem Ablauf ist der Clipapplikator bereit, den nächsten Zyklus zu starten.

Bewährt haben sich für das Außenrohr Durchmesserwerte von 5,5 mm, 10 mm oder 12 mm jeweils ± 10 % zu wählen und eine Länge von 318 mm vorzugeben, wobei dabei eine Varianz von ± 50 % sinnvoll erscheint.

Die Zunge, bzw. eine Zungenlasche, ist durch mehrere Klammern ohne eine Führung derselben zu übernehmen hindurchgeführt. Es ist als langgestrecktes Element mit hoher Knickneigung zum Vorschieben und Führen des am weitesten distal gelegensten Clips mit Toleranz ausgebildet, wobei jedoch ein Knicken durch eine längsseitige Bördelung und/oder angebrachte Längssicken verhindert wird. Die Zunge stellt ein sicheres Greifen der Klammer / des Clips sicher. Sie überträgt Kräfte auf die Klammer bei einem Winkel- und einem Lagewechsel. Sie ist zum Abfangen von Toleranzen mit einer flexiblen Spitze, etwa aus dünnerem Material, oder etwa mit Elastizitätslöchern, etwa in Form von Ausschnitten zur Verringerung der Biegequerschnitte versehen. Ein Aushebeln der Drucklaschen / Schiebekanten ist durch eine Freihaltung in Form eines Ausschnittes auf der Oberseite der Zungenspitze verhindert. Durch ein Überragen des Bleches an der Zungenspitze und eine Ablage auf der Innenseite der Klammer-Schenkelkehle, wird ein Gewebeschutz erreicht und ein Abrutschen der Zunge vom Klammersteg verhindert. Dieses überragende Blechelement wird als Abstandshalter oder Überstand bezeichnet.

Ein Vorschubverteiler sorgt für eine direkte mechanische feste Verbindung zum Antriebsstrang. Dies ermöglicht einen Vorschubhub bzw. einen Förderhub. Der Vorschubverteiler hat eine direkte mechanische feste Verbindung zur Zunge. Er hat auch eine direkte mechanische, aber lose, d.h. in einer Richtung verschiebliche, Verbindung zur Förder- und Mitnahmeschiene. Dadurch wird ermöglicht, eine Linearbewegung in zwei oder mehr Beträge aufzuteilen. Es ist hilfreich, einen Nocken zu nutzen, der verschieblich in ein Langloch greift.

Ein Wegfall des Vorschubaufteilers ist möglich, wobei dann auf eine Integration in die Zunge zurückgegriffen werden sollte. Sowohl die Rückhalteschiene, als auch die Förder- und Mitnahmeschiene / Vorschubschiene, weist Laschen auf. Diese dienen einem sicheren Griff der Klammern bei wenig Schlupf bzw. geringer Toleranz. Eine geringe Reibung ist dabei wünschenswert. Sie dienen der Führung / Stützung von Bauteilen, wie etwa der Zunge. Sie dienen der Führung der Klammern und definieren eine Clipführungsbahn, welche die axiale Lage der Klammern vorgibt, wobei die Clipführungsbahn ausschließlich durch die Rohrinnenwand und die Oberseite des Rückhaltebleches bestimmt ist. Die Führung des Vorschubbleches wird durch die Innenwand des Außenrohres und das durch die Klammern gebildete Paket gebildet oder bei Aufbrauch der Klammern durch die Zunge, welche sich wiederum auf der Rückhalteschiene abstützt, bewirkt. Es ist auch ein Tauschen der Lage der Bleche denkbar: Eine Ablage der Clips auf dem Vorschubblech und der Abstützung an der Rohraussenwand. Ist plausibel.

Es werden dabei folgende Funktionen erfüllt:

- Steuerung der Elastizität durch die Geometrie der Laschen (Minimierung der Reibkräfte) und
- Steuerung der Führungs- und Halteaufgaben durch die Geometrie / Höhe der Laschen.

Für die erste Steuerungsfunktion wird auf die Parameter Blechdicke, Laschen- und Ausschnittsbreite, Lage von Sicken und Knickungen, Geometrie von Biegungen und Orte von Biegelinien, sagittale Geometrie der Laschen, frontale Geometrie und axiale Geometrie sowie auf das Einbringen von Ausschnitten, etwa über Elastizitätslöcher, zurückgegriffen. Für den zweiten Punkt wird auf eine individuell eingestellte Laschengeometrie, eine Halterung und Abstützung der Zunge durch mindestens eine Lasche, und die Haltung und Abstützung des Blechs auf der Zunge mit mindestens einer Lasche abgestellt. Dabei hat jede Lasche eine spezifische Aufgabe. Einige Laschen können höher sein als die anderen, um die Zunge zu stützen. Nicht alle Laschen sind also gleich hoch. Dadurch wird die Reibung minimiert. Es sollen steife Leit- und Förderelemente in Längsrichtung erreicht werden, die hier elastisch in Querrichtung ausgestaltet sind.

Zur Brücke an der Rückhalteschiene ist noch auszuführen, dass an der Rückhalteschiene die Funktion für eine Clipbahn / Klammerleitbahn vorgehalten ist. Sie ist so ausgeprägt, dass sich die Lasche auf dem oberen Maul abstützt und von diesem bei einer Verschlussbewegung verdrängt wird.

Es gibt verschiedene Arten die Rückhalteschiene am Außenrohr zu fixieren. Etwa durch radial nach innen ragende Vorsprünge am Außenrohr, durch geeignete Laschen und Ausschnitte in der Rückhalteschiene und/oder Laschen / Vorsprünge und Ausschnitte am Außenrohr, vorzugsweise so, dass beim Rückhub eine Zugkraft auf die Rückhalteschiene wirkt, damit die Rückhalteschiene keinerlei Knicklast ertragen muss. Eine Fixierung der Rückhalteschiene direkt am Federgegenhalter ist möglich. Ein Versatz der Fixierung (Links-rechts-Asymmetrie) um die Steifigkeit zu erhöhen und zum Unterbinden einer direkten Biegelinie, ist ebenfalls wünschenswert. Die Gestaltung der Rückhalteschiene mit Federelementen, welche diese nach oben an die Clip Line / Klammerlinie drückt, wirkt als Toleranzausgleich.

Es gibt auch Maßnahmen zur Versteifung des Blechs für eine Kraftübertragung mit geringer Ausknickneigung. Dazu bieten sich geschickt platzierte Sicken und ein definierter Randkragen an. Das Außenrohr hat Halteaufgaben, wie das Befestigen des Mauls, das Befestigen der Rückhalteschiene, das Befestigen eines Vorschubapparates. Es ist vorzugsweise ein einstückiges, einteiliges Bauteil mit kurzen Toleranzketten. Es kann in einem einzigen Schnittvorgang in einer einzigen Aufspannung, bei hoher Genauigkeit geschaffen werden.

Von einem laschenfreien Rohrabschnitt wird eine Führungsaufgabe übernommen, nämlich die des Führens der Klammern an der Rohrwand, insbesondere unter Berühren der Rohrwand. Ein Führen der Bleche, des Schubrohres, des Vorschubverteilers und des Rückhaltebleches wird ebenfalls erreicht.

Die erwähnten Laschen dienen der Befestigung bspw. eines Rückhaltebleches und sind zur Lagefixierung einsetzbar. Sie sind elastisch in der Höhe, aber auch seitlich elastisch. In Axialrichtung gesehen haben sie eine kleine Toleranz. Aus einer Richtung sind sie (einseitig) fügbar. Sie sind mittels einem (einzigen) Schnitt und einer Biegung herstellbar.

Die Erfindung wird nachfolgend mit Hilfe einer Zeichnung näher erläutert, in der unterschiedliche Ausführungsbeispiele dargestellt sind. Es zeigen:

Fig. 1      eine perspektivische Darstellung eines Zusammenbaus eines erfindungsgemäßen medizinischen Schaftinstruments mit einem Griff,

Fig. 2      eine Explosionsdarstellung des medizinischen Schaftinstruments aus Fig. 1 in perspektivischer Darstellung ohne den in Fig. 1 enthaltenen Griff,

Fig. 3      den Zusammenbau der Einzelteile aus Fig. 2 in perspektivischer Darstellung,

Fig. 4 bis 6      perspektivische Darstellungen nur der distalen Spitze des medizinischen Schaftinstruments aus Fig. 1 ohne das Außenrohr,

Fig. 7      einen Querschnitt durch einen Instrumentenschaft des Schaftinstruments aus Fig. 1,

Fig. 8      ein Diagramm zur Darstellung des Kraftverlaufs aufgrund von Reibung beim Überfahren einer von dem Schaftinstrument aus Fig. 1 applizierbaren Klammer über ein einzelnes wegklappbares Formschlußelement, wie eine Rückhaltelasche oder ein Klammer-Mitnahmeelement,

Fig. 9      ein Diagramm zur Darstellung des Kraftanstiegs beim "Aufsammeln" der Klammern / Clips während des Vorschubs, bedingt durch einen Förderhub und während eines Rückhubs, zurückgehend auf Reibung beim Überfahren der Laschen durch die Klammern,

Fig. 10      eine vergrößerte perspektivische Darstellung eines Abschnitts einer Rückhalteschiene mit Befestigungsvorsprüngen und Rückhaltelaschen,

Fig. 11      eine weitergehende Vergrößerung einer Rückhaltelasche aus Fig. 10,

Fig. 12      eine Ansicht von oben auf die Rückhaltelasche der Fig. 10 und 11,

Fig. 13      das Anliegen einer Rückhaltelasche an einer als Doppelsteg-Clip ausgestalteten Klammer, und zwar auf deren proximalen Seite,

Fig. 14      den Zustand einer Rückhaltelasche beim von einer Klammer schon überfahrenen und wieder freigegebenen Stellung,

Fig. 15      eine Nachstellung einer Rückhaltelasche in einer noch überfahrenen und nicht von einer Klammer freigegebenen Position,

Fig. 16      das Anliegen einer Klammer an einer ausgestellten und ausgeformten Rückhaltelasche als Abstützung am Klammerrücken / Cliprücken zum Verhindern eines Rückfahrens der Klammer,

Fig. 17      den Zusammenbau einer Rückhalteschiene, auf der eine Vielzahl von Klammern in Lagerpositionen gelagert sind, welche durch eine Zunge durchdrungen sind, wobei die Klammern von oben von einer Förder- und Mitnahmeschiene kontaktiert sind,

Fig. 18    ein Längsschnitt durch die Elemente aus Fig. 17, bei der ein sandwichartiges Blechpaket und ein Clipstapel / Klammerstapel mit gegenseitiger Abstützung über Laschen bewirkt ist,

Fig. 19    eine perspektivische Darstellung auf eine erste Ausführungsform eines Bauteils zur Hubaufteilung, nach Art eines Vorschubaufteilers,

Fig. 20    eine gedrehte perspektivische Darstellung der Bauteile aus Fig. 19,

Fig. 21    ein Längsschnitt durch die Bauteile aus Figur 19 und 20,

Fig. 22    eine zweite Ausführungsform zum Erreichen einer Hubaufteilung, bei integrierter Zunge,

Fig. 23    einen Längsschnitt durch die Ausführungsform aus Fig. 22 im Bereich eines Vorschubaufteilers,

Fig. 24    eine ausgebrochene perspektivische Darstellung eines Zusammenbaus aus einem Außenrohr, und einem Rückhalteblech in einer montierten Position,

Fig. 25    die Kombination der Bauteile aus Fig. 24 in einer Stellung bei Beginn der Montage,

Fig. 26    die Bauteile der Fig. 25 und 26 zu einem Zeitpunkt, bei der die Montage bereits fortgeschritten ist,

Fig. 27    die Bauteile der Fig. 25 zum Zeitpunkt des Abschlusses der Montage, wie in Fig. 25 gezeigt, jedoch vergrößert,

Fig. 28    ein weiteres Ausführungsbeispiel zum Fixieren des Rückhalteblechs über einen Federgegenhalter an einem nicht dargestellten Außenrohr durch Nutzung von Ausschnitten,

Fig. 29    eine distale Spitze des Rückhaltebleches mit einem / einer spitzennah gelagerten Clip / Klammer,

Fig. 30    eine gedrehte Darstellung der Bauteile aus Fig. 29,

Fig. 31    eine Ansicht von oben zwischen zwei Maulteil-Branchen, die einen Maulteilbereich bilden, wobei eine distale Spitze des Rückhalteblechs als (Teil-) Deckel nach Art einer Auflageplatte oder Brücke dient,

Fig. 32    eine zur Fig. 10 ähnliche Darstellung im Bereich von als Montagelaschen agierenden Vorsprüngen, die eine asymmetrische Biegelinie vorgeben und eine direkte Biegelinie vermeiden,

Fig. 33    eine Unterseitendarstellung des Rückhalteblechs in perspektivischer Form zur Visualisierung von dort vorhandenen Federlaschen,

Fig. 34    einen distalen Endbereich einer Zunge / Vorschubzunge mit einem als Eintauchöffnung agierendem Ausbruch,

Fig. 35    eine Ansicht in perspektivischer Form von unten auf die distale Spitze der Zunge aus Fig. 34 mit einer Leitsicke nach Art eines Abweisers, die als Bewegungsleitelement agiert und biegesteifigkeitserhöhend wirkt,

Fig. 36    eine Teildarstellung der Klammern, des Rückhalteblechs und der Zunge im Bereich der Leitsicke,

Fig. 37    eine Darstellung der Bauteile aus Fig. 36 in etwas gedrehter Form,

Fig. 38    eine Darstellung auf die Bauteile aus Fig. 36 und 37 in perspektivischer Form von hinten,

Fig. 39    den Eingriff der Zunge an der distalsten Klammer,

Fig. 40    die Bauteile aus Fig. 39 in einer Darstellung perspektivisch von hinten,

Fig. 41    eine Vergrößerung des Kontaktbereichs zwischen der Klammer und einer Schiebekante / Drucklasche der Zunge,

Fig. 42      die Klammer beim Eintauchen in die Eintauchöffnung der Zunge in einer Darstellung perspektivisch von oben,

Fig. 43      eine Klammer beim Zuführen entlang der Zunge zum distalen Spitzenbereich der Zunge,

Fig. 44      der Federgegenhalter in perspektivischer Darstellung,

Fig. 45      ein Querschnitt durch den Federgegenhalter im Bereich von Dichtflächen,

Fig. 46      eine perspektivische Darstellung des Federgegenhalters der Fig. 44 und 45 von unten,

Fig. 47 und 48      eine Seitenansicht und eine Vorderansicht des Federgegenhalters der Fig. 44 bis 46,

Fig. 49      eine Seitenansicht des Federgegenhalters der Fig. 44 bis 48 bei an ihm angebrachtem Rückhalteblech und gekoppelter Förder- und Mitnahmeschiene,

Fig. 50      der Federgegenhalter, wie er in ein Rückhalteblech eingeclipst ist, und

Fig. 51      eine Darstellung der Bauteile aus Fig. 50 mit Blick auf eine Freihaltung (Langloch mit Hubbegrenzung) für den Vorschubaufteiler bei in den Federgegenhalter eingeclipsten Rückhalteblech.

Die Figuren sind lediglich schematischer Natur und dienen ausschließlich dem Verständnis der Erfindung. Die gleichen Elemente werden mit denselben Bezugszeichen versehen. Merkmale der einzelnen Ausführungsbeispiele können untereinander ausgetauscht werden. Solche Merkmale sind also untereinander austauschbar.

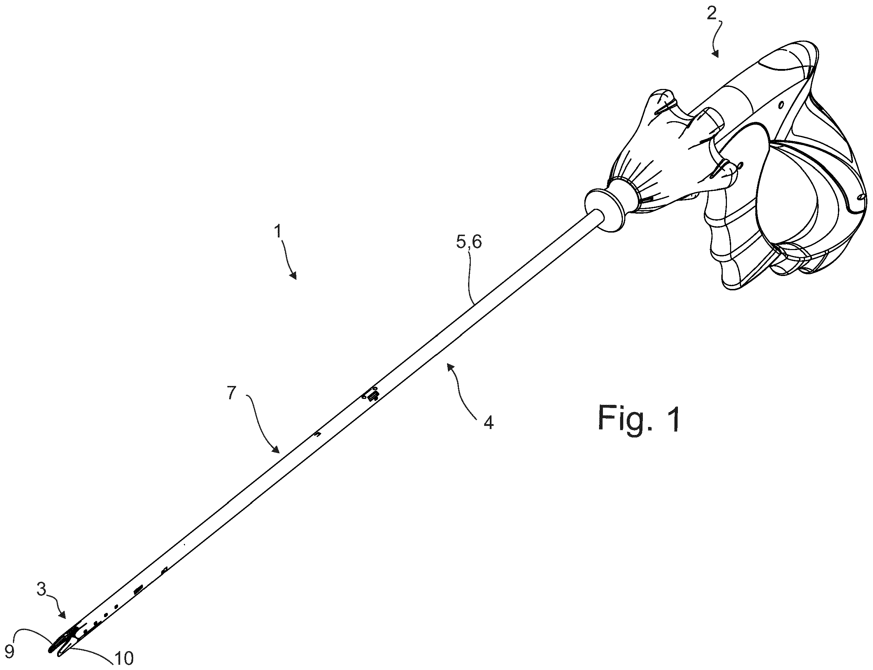

In Fig. 1 ist eine erste Ausführungsform eines medizinischen Schaftinstrumentes 1 dargestellt. Es weist einen Instrumentengriff 2 an seinem proximalen Ende auf. Der Instrumentengriff 2 kann auch kurz als Griff bezeichnet werden. Der Griff kann nach Art eines "Challenger-Griffes" ausgebildet sein. Am distalen Ende des medizinischen Schaftinstrumentes 1 ist ein Instrumentenkopf 3 ausgebildet. Zwischen dem Instrumentengriff 2 und dem Instrumentenkopf 3 ist ein Instrumentenschaft 4 angeordnet, welcher die beiden Bauteile miteinander verbindet.

Der Instrumentenschaft 4 besitzt außenseitig ein Außenrohr 5. Das Außenrohr 5 kann einen Kreisringquerschnitt aufweisen und nach Art eines Hohlzylinders ausgeformt sein. Der als Kontaktbereich für eine Hand agierende Instrumentengriff 2 gibt einen Befehl eines Operateurs an den Instrumentenschaft 4 weiter, um mit Hilfe dessen den Instrumentenkopf 3 zu betätigen. Innerhalb des Außenrohres 5, das als Gehäuse 6 agiert, ist ein Clipmagazin 7 vorhanden. Das Clipmagazin 7 ist ein Magazin zum Lagern von Klammern, Clips, Klipsen oder anderen für einen Ligatur-Einsatz geeignete Klemmenausgestaltung. Solche Klammern, insbesondere Ligatur-Klammern oder -Klemmen sind zur plastischen Verformung oder zur Verrastung vorgesehen, um im verformten Zustand ein Organ eines Säugetiers, wie ein Blutgefäß eines Menschen abzubinden.

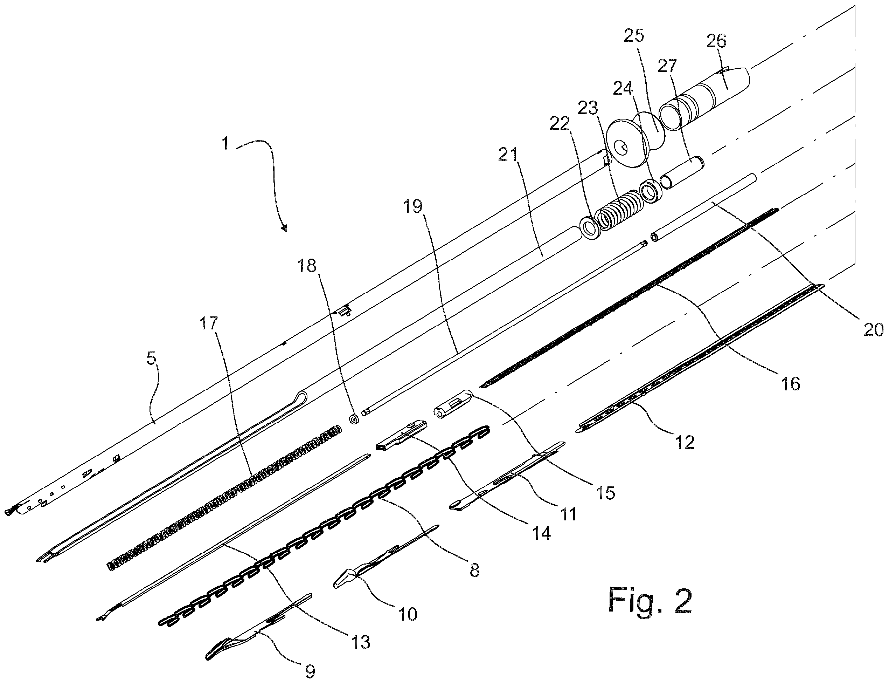

Eine Vielzahl solcher Klammern 8 ist in der Fig. 2 wiedergegeben.

In Fig. 2 sind die Einzelteile des medizinischen Schaftinstrumentes 1, mit Ausnahme des Instrumentengriffs 2 wiedergegeben. Insbesondere sind ein oberes Maulteil / eine obere Maulteilbranche 9, ein unteres Maulteil / eine untere Maulteilbranche 10, ein Schieber 11, der als Nockenträgerbauteil bezeichnet werden kann und eine Rückhalteschiene 12 zu erkennen.

In der dort dargestellten Ausführungsform sind in Summe 20 Klammern 8 eingesetzt. Es können aber auch mehr oder weniger dieser Klammern 8 verwendet werden. Eine Zunge 13, die auch als Vorschubzunge bezeichnet werden kann, ein Vorschubaufteiler 14, ein Federgegenhalter 15 und eine Förder- und Mitnahmeschiene 16, die auch als Vorschubschiene bezeichnet werden kann, ist ebenfalls enthalten. Ferner ist eine Vorschubfeder 17, eine Dichtscheibe 18, eine Vorschubstange / Schubstange 19 und ein Vorschubstangenendstück 20 eingesetzt. Ein Schubrohr 21 schließt an einen Dichtring 22 an. Der Dichtring 22 ist eine distale Begrenzung einer Druckfeder 23, die an einen Federflansch 24 grenzt. Die Druckfeder 23 stützt sich am Federflansch 24 ab. Die Druckfeder 23 ist für ein Rückstellen der Maulteile 9 und 10 zuständig, also für das Bewegen des oberen Maulteils 9 vom unteren Maulteil 10 weg.

Die Bauteile 9 bis 24 sind zum Einsatz innerhalb des Außenrohres 5 vorgesehen. Das Außenrohr 5 steckt nach der Montage in einem Griffflansch 25. Der Griffflansch 25 seinerseits ist in kraft-, form- und/oder stoffschlüssigen Kontakt mit einem Griffstück / Griffankoppelbauteil 26, um den Instrumentengriff 2 zu koppeln. Proximal des Federflansches 24 ist auch ein Hohlzylinderendstück 27 angeordnet. Dieses Hohlzylinderendstück 27, wie auch das Vorschubstangenendstück 20 sind aus dem Griffstück 26 proximal herausragend zu erkennen.

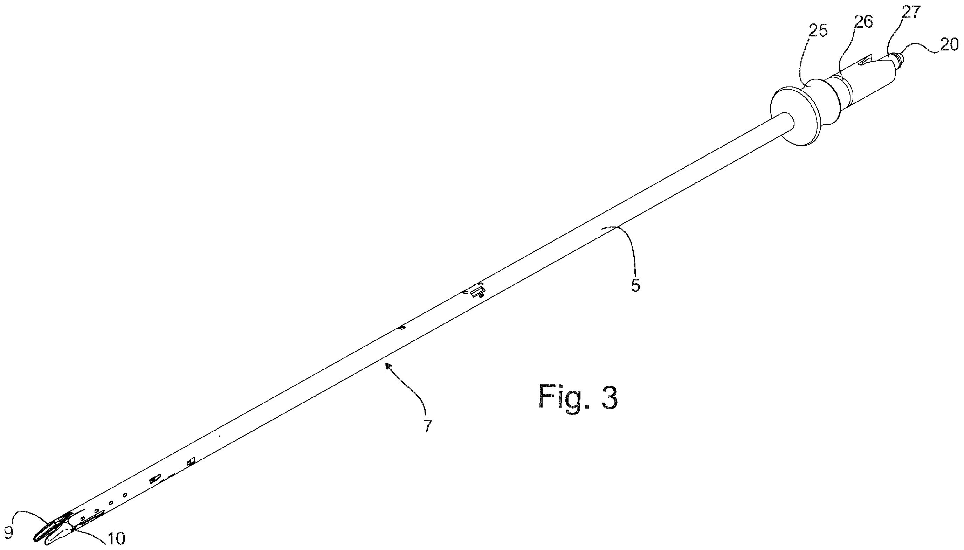

In der Fig. 3 sind die aus Fig. 2 bekannten Komponenten im zusammengebauten Zustand dargestellt.

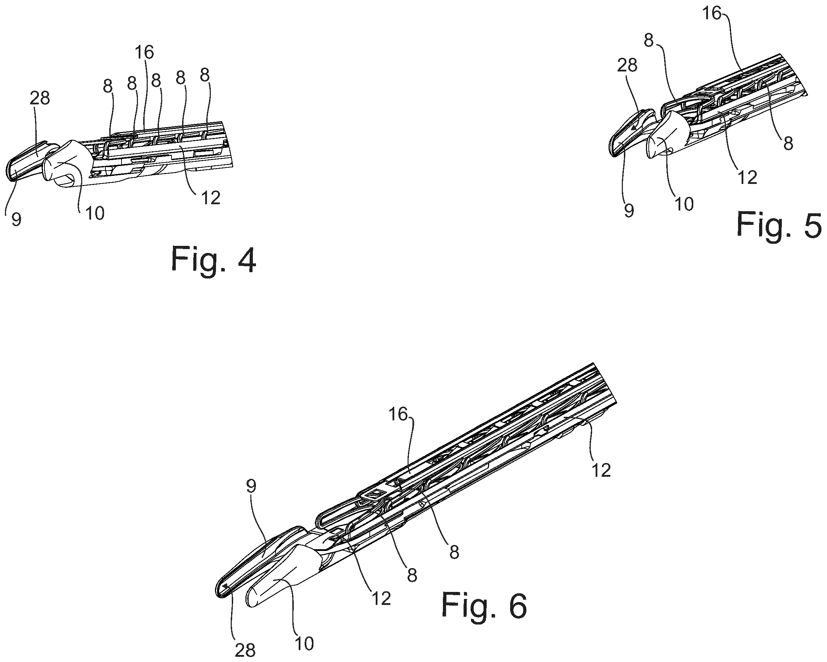

In den Fig. 4 bis 6 ist der Zusammenbau der oberen Maulteil-Branche 9 mit der unteren Maulteil-Branche 10 zur Aufnahme der distalsten Klammer 8 innerhalb einer Schalenform 28 angedeutet. Eine als Rückhalteblech ausgebildete Rückhalteschiene 12 ist unterhalb der Förder- und Mitnahmeschiene 16 angeordnet.

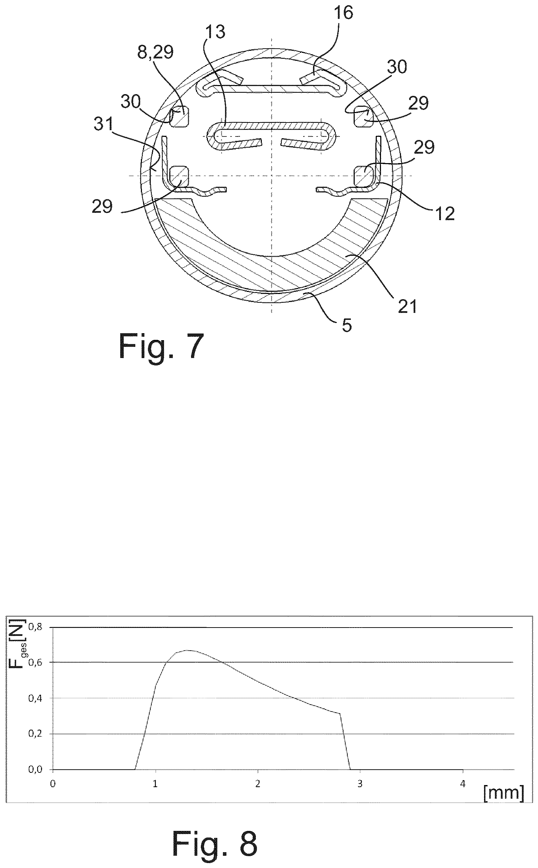

In Fig. 7 ist die Führung der Klammern 8 im Querschnitt gut zu erkennen. So stützt sich die dort dargestellte Klammer 8 mit ihren vier Klammerstegen 29 sowohl an der Rückhalteschiene 12, als auch dem Außenrohr 5 ab. Die Klammerstege 29 können auch verkürzt als Stege bezeichnet werden.

Dabei weist das Außenrohr 5 Berührungsflächen / Anlageflächen 30 zum Kontaktieren der oberen Klammerstege 29 der Klammer 8 auf. Die Klammer 8 ist dabei nach Art eines Doppelsteg-Clips ausgestaltet. Die Zunge 13 ist zum Ausstoßen der vordersten, ersten Klammer 8, d.h. der distalsten Klammer 8 vorgesehen, wohingegen die Förder- und Mitnahmeschiene 16, nach Art eines Vorschubbleches zum Bewegen aller Klammern 8 im Clipmagazin 7 vorgesehen ist. Die Berührungsflächen / Anlageflächen 30 sind so gestaltet, dass sie eine Gleitbewegung der Klammern 8 an ihr entlang ermöglichen.

Möglich, aber nicht dargestellt, ist es, dass im Bereich der zum Kontaktieren der Berührungsfläche 30 vorgesehenen Klammerstege 29 in dem Außenrohr 5 Ausnehmungen, wie Schlitze, Nuten, Riefen oder Durchgangslanglöcher ausgebildet sind, durch welche die Klammern 8 nach außen, auf die Außenseite des Außenrohres 5, das Außenrohr 5 durchdringend, hindurchragen können. Auf diese Weise kann eine besonders kompakte Ausgestaltung des Schaftinstrumentes 1 erreicht werden.

Die Klammern 8 stützen sich auf der Rückhalteschiene 12 mit ihren Klammerstegen 29 ebenfalls ab, und zwar derart, dass im Zusammenwirken mit dem Abstützen an dem Außenrohr 5 eine Kompression / Verbiegung der Klammern 8 erzwungen ist. Die Klammern 8 berühren sich dabei nicht. Die Zunge 13 ist durch die doppelstegclipartigen Klammern 8 hindurchgeführt. Eine Art Auffädelung der Klammern 8 ist die Folge.

Die Klammerstege 29 bilden dabei Schenkelabschnitte. Der Vorschub der Klammern 8 wird durch eine Vor- und Rückbewegung eines langgestreckten Bauteils mit Laschen, nach Art von Vorsprüngen, Lamellen oder Widerhaken bewirkt. Die Klammern 8 sind ausschließlich an einer Innenwandung 31 des Außenrohres 5 und einer blechartigen Rückhalteschiene 12 geführt. Dadurch wird der Bauraum effizient genutzt. Geräusche werden verhindert, insbesondere ein Klappern. Ein präzises Führen ist die Folge. Auch ist ein Toleranzausgleich erreicht. Es wird kein extra Kanal benötigt.

Ein kanalartiger Aufbau wie in Fig. 7 gezeigt, reicht dabei aus, wobei die Verwendung von Blechen Vorteile bei der Steifigkeitsauslegung hat.

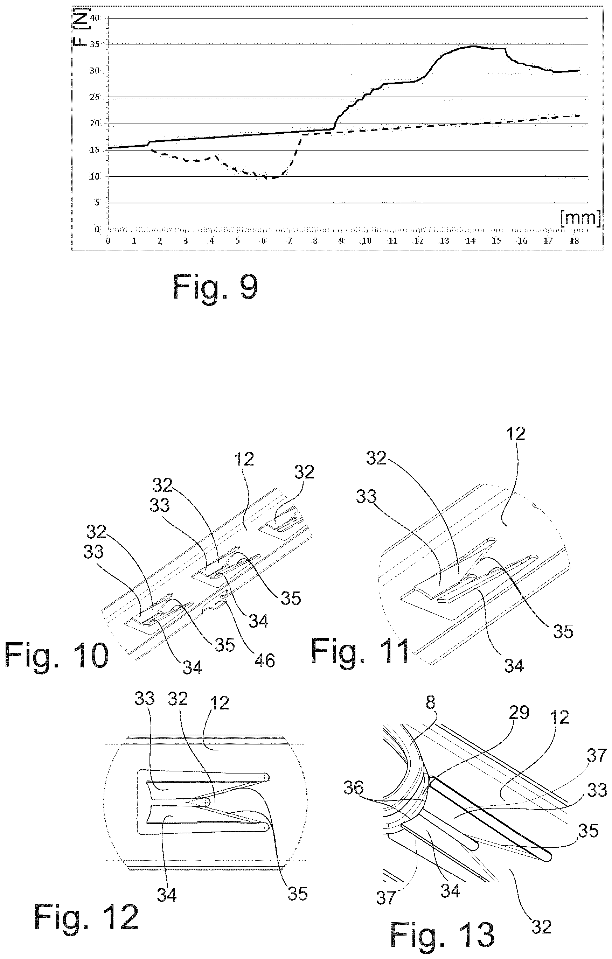

Die Rückhalteschiene 12 weist Rückhaltelaschen 32 auf. Diese sind bspw. gut in den Fig. 10 bis 16 zu erkennen. Dort ist auch eine schmetterlingsartige Aufteilung der Rückhaltelaschen 32 in einen ersten Rückhaltelaschenabschnitt 33 und einen zweiten Rückhaltelaschenabschnitt 34 zu erkennen. Diese beiden Rückhaltelaschenabschnitte 33 und 34 formen somit eine Art Schmetterlingslasche. Die Flügel einer Schmetterlingslasche können auch als erste und zweite Rückhaltelaschenabschnitte 33 und 34 bezeichnet werden.

Über die Rückhaltelaschenabschnitte 33 und 34 gleiten / rutschen dann die Klammerstege 29, die auch als Schenkel bezeichnet werden können, hinweg und bewirken ein Verklappen der Rückhaltelaschenabschnitte 33 und 34 entlang einer Schwenk- oder Biegelinie 35. Die Schwenk-Biegelinie 35 kann auch als Biege- oder Schwenkachse bezeichnet werden.

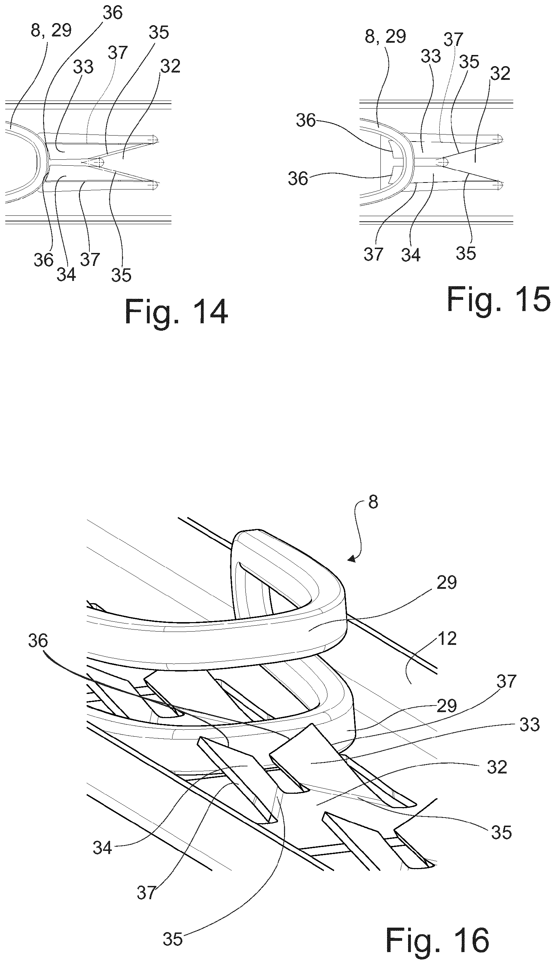

Wie besonders gut in den Fig. 13 und 16 zu erkennen, ist ein Bewegen der Klammern 8 in Richtung proximal durch das Anliegen eines proximalen Endes der Klammer 8 an einer distalen Kante 36 der Rückhaltelasche 32 verhindert. Wie in den Fig. 10 und 16 gut zu entnehmen ist, liegt also eine schmetterlingsartige Laschenform vor. Abstehende Laschen mit Flügeln, die eine größtmögliche Kante / Fläche zum Abstützen der Klammern 8, am Klammerrücken aufweisen, sollen dabei eine große Steifigkeit bieten. Die Rückhaltelaschengeometrie ist so angelegt, dass sie beim Überfahren ausweichen kann, ohne das Blechniveau zu unterschreiten. Die Rückhaltelaschen 32 legen sich also flach. Im aufgestellten Zustand bilden Seitenkanten 37 der Rückhaltelaschen 32 nahezu parallele Linien. Sie laufen also vornehmlich in Längsrichtung. Zumindest ein Winkel von proximal her größer als 0° ist akzeptabel. Dies sorgt für eine minimale Reibung. Beim Überfahren der Rückhaltelasche 32 von proximal her, durch die Klammer 8, vergrößert sich dieser Winkel.

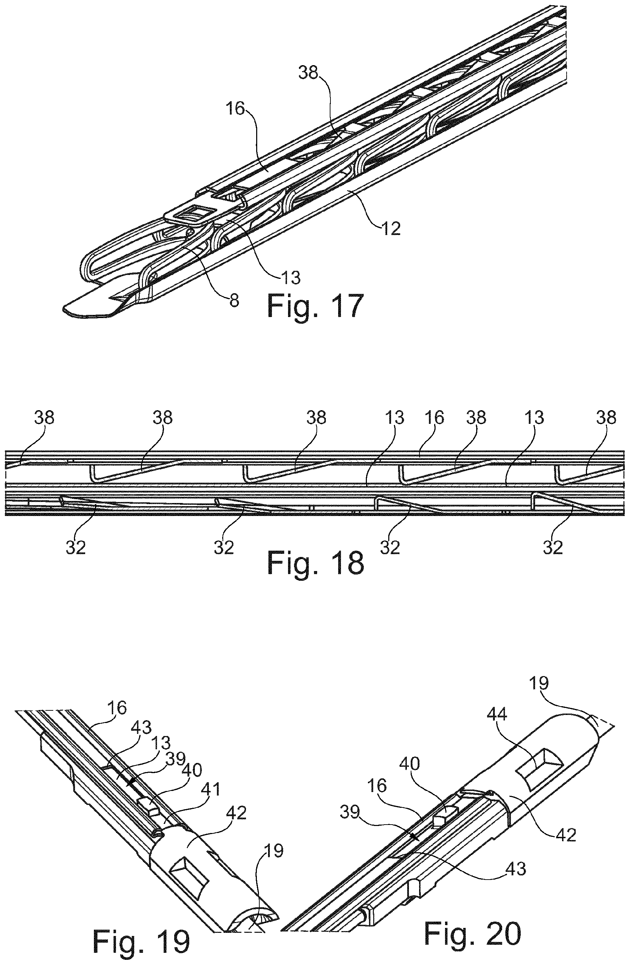

Unter Vorgriff auf Fig. 18 sei erläutert, dass auch die Förder- und Mitnahmeschiene 16 Laschen aufweist, nämlich Klammermitnahmeelemente bzw. Klammermitnahmelaschen 38. Lascheninkremente und Laschenabstände sind zur Bauteillängenbegrenzung, insbesondere für die Magazinlänge bedeutsam. Sie steuern die Reibarbeit. Sie sind geschickt bzgl. ihres Ortes in Relation zum Vorschubhub, der auch als Förderhub bezeichnet werden kann, zum Positionieren. Sie lassen sich einfach ausformen, wenn ein geformter Blechstreifen oder ein Kunststoffbauteil als Ausgangsbasis für sie genutzt wird. Sie sollen vorzugsweise nach Art von federbaren Laschen mit genau definierten Abständen angeordnet sein. Die Abstände der Rückhaltelaschen 32 zueinander kann aber über die Länge des Bleches variabel sein, d.h. nicht konstant gehalten sein. Die Abstände der Klammer-Mitnahmeelemente / -laschen 38 zueinander soll ebenfalls über die Länge des Bleches variabel sein, d.h. nicht konstant bleiben.

Die Abstandsinkremente der Laschen sollen so gewählt sein, dass aus einer Ruhelage der Klammern 8 ein sequentielles Einsammeln der Klammern 8 erfolgt, was bei nichtkonstanten Abständen die Folge ist. Dadurch wird ein stetiger Kraftanstieg vollzogen. Ein vorbestimmter Betrag an aufzubringender Kraft wird dann auch nicht überschritten.

Die Abschnittsinkremente der Laschen soll so gewählt sein, dass die Magazinlänge minimiert wird. Die Abstandsinkremente der Laschen sind dabei so gewählt, dass ein sequentielles Aufsammeln von distal nach proximal, abhängig von der Ruhelage der Klammern, stattfindet, um ein Aufschieben der Klammern aufeinander zu vermeiden, bzw. diesem entgegen zu wirken. Zur Ermittlung des Abstandes A bietet sich folgender Zusammenhang an:

Als Abstand (A) wird der Abstand zwischen der j-ten und der (j-1)-ten Lasche entweder der Rückhalteschiene 12 oder der Vorschubschiene / Förder- und Mitnahmeschiene 16 bezeichnet, wobei j die Position der Lasche von distal aus angibt. n entspricht der Gesamtanzahl der Clips im Applikator.

Abstand Rückhaltelasche (ARHL) = konstanter Abstand (AK) + inkrementeller Abstand RHL (AjRHL)

inkrementeller Abstand RHL (AjRHL) = Inkrement RHL IKRHL • (n-j)

Abstand Vorschublasche (AVSL) = konstanter Abstand (AK) + Konstante (K) + inkrementeller Abstand VSL (AjVSL)

inkrementeller Abstand VSL (AjVsL) = Inkrement VSL IKVSL • (n-j)

Allerding ist der konstante Abstand (AK) abhängig von den Klammern, deren Größe und deren Geometrie, liegt also beispielsweise bei etwa 8,5 mm. Möglich ist es auch, ein konstantes Inkrement (iK) und ein variables Inkrement (iV) vorzuhalten. Das Gesamtinkrement I berechnet sich dann wie folgt: I = IK + IV. Damit lässt sich der Ort der auftretenden Maximalkraft und somit das Ausknicken des Bleches steuern.

In Fig. 8 ist das Reibverhalten in Abhängigkeit von der Überfahrposition der Klammer 8 über eine der Laschen 32 und 38 wiedergegeben. Auf der Abszisse ist die Laschenlänge in mm angetragen, wohingegen auf der Ordinate die Gesamtkraft $F_{ges}$ in Newton angegeben ist.

In Fig. 9 ist eine Darstellung des Kraftanstiegs beim "Aufsammeln" der Klammern 8 während einerseits eines Förderhubes und andererseits während eines Rückhubes aufgrund der Reibung beim Überfahren aller Laschen (Rückhaltelaschen 32 und Klammer-Mitnahmelaschen 38) mit allen Klammern 8 wiedergegeben. Dabei ist mit der durchgezogenen Linie der Kraftanstieg / Kraftverlauf während des Vorschubes / Förderhubes und mit der gestrichelten Linie der Kraftanstieg / Kraftverlauf während des Rückhubes wiedergegeben. Auf der Abszisse ist der Vorschubweg in mm angetragen, wohingegen auf der Ordinate der Kraftbedarf in Newton angetragen ist.

Zurückkommend auf die Fig. 17 und 18 sei auf die sandwichartige Anordnung der Förder- und Mitnahmeschiene 16 oberhalb der Zunge 13, ihrerseits oberhalb der Rückhalteschiene 12 hingewiesen. Die Rückhalteschiene 12, die Förder- und Mitnahmeschiene 16 und die Zunge 13 sind also übereinander axial verschieblich angeordnet. Die Laschenhöhen wenigstens einer Anzahl der Laschen 32 und/oder 38 sind dabei so bemessen, dass sie die in der Mitte befindliche Zunge 13 führen.

In Anbetracht der Fig. 19 bis 21 sollte klar werden, dass es wünschenswert ist, eine Vorschubaufteilung mittels eines Langloches 39, in das ein Nocken 40 greift, zu erreichen. Der Nocken 40 steht von einem Vorschubaufteiler 41 ab, welcher separat von einem Federgegenhalter 42 ausgestaltet sein kann. Der Nocken 40 kann ein integraler Bestandteil des Federgegenhalters 42 sein. Jedenfalls greift die Vorschubstange 19 durch den Federgegenhalter 42 hindurch und ist axialfest mit dem Vorschubaufteiler 41 verbunden. Der Nocken 40 des Vorschubaufteilers 41 greift dann in das Langloch 39 der Förder- und Mitnahmeschiene 16 hindurch. Die Zunge 13 ist axialfest mit der Vorschubstange 19 verbunden, so dass eine von der Vorschubstange 19 übertragene Bewegung direkt an die Zunge 13 weitergegeben wird und erst, wenn der Nocken 40 auf eine Anschlagkante 43 trifft, an die Förder- und Mitnahmeschiene 16 weitergegeben wird. Es sind somit zwei ineinandergreifende Bauteile so ausgebildet, dass ein Zapfen, Nocken oder ein anderweitiger Überstand, in ein Loch, eine Nut oder eine Aussparung so eingreift, dass eine axiale Relativbewegung der beiden Teile zueinander um einen bestimmten Betrag zugelassen wird, jedoch nach Erreichen eines Anschlags eine gemeinsame Bewegung bewirkt wird.

Wie in den Fig. 19 und 20 besonders gut zu erkennen ist, weist der Federgegenhalter Ausnehmungen 44 auf, in die Vorsprünge oder Laschen des Außenrohres 5 eingreifen können, um eine axiale Sicherung zu bewirken.

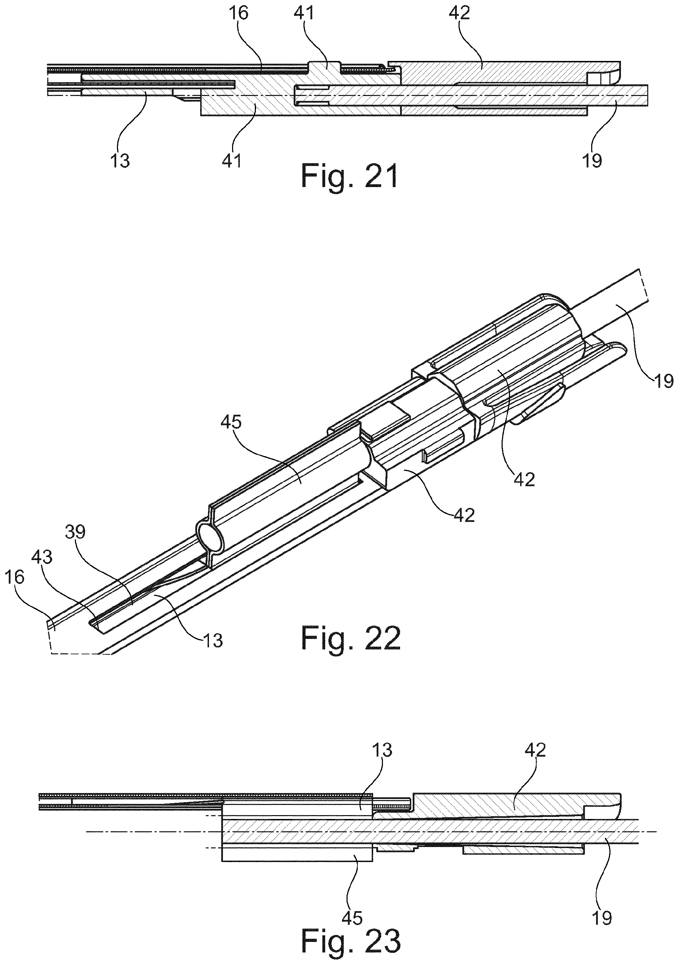

In den Fig. 22 und 23 ist eine davon unterschiedliche Variante gezeigt, nämlich bei der die Schubstange 19 direkt mit der Zunge 13 verbunden wird. Dazu wird die Zunge 13 um ein distales Ende der Schubstange 19 nach Art eines Falzbleches herumgebördelt. Natürlich ist es auch möglich, dass die Zunge 13 ein einstückiger Bestandteil der Schubstange 19 ist. Bei der Ausgestaltung der Fig. 22 und 23 gibt es keinen separaten Vorschubaufteiler 41. Allerdings weist jenes die Verbindung zwischen der Zunge 13 und der Schubstange 19 sicherstellende Falzblech 45 auch vertikal durch ein in der Förder- und Mitnahmeschiene 16 enthaltenes Langloch 39, um ähnlich wie in dem zuvor beschriebenen Ausführungsbeispiel in Anschlag zu gelangen mit einer Anschlagkante 43, um die Förderhubinitiierung an der Förder- und Mitnahmeschiene hervorzurufen. Während in der Fig. 22 eine perspektivische Ansicht vornehmlich von unten dargestellt ist, ist in der Fig. 23 ein Längsschnitt dargestellt. Es ist möglich, dass die Zunge 13 mit der Schubstange 19 verklebt, verschweißt oder vercrimpt ist. Letztlich werden zwei sehr präzise Anschläge zur Verfügung gestellt, wodurch ein genaues Arbeiten mit dem medizinischen Schaftinstrument 1 ermöglicht wird.

Die Rückhalteschiene 12 hat nicht nur eine rückhaltende Funktion für die Klammern 8, nämlich die Verhinderung des Rücklaufs der Klammern 8 beim Rückhub in die Neutralposition der Förder- und Mitnahmeschiene 16, sondern bildet im Querschnitt auch die untere Begrenzung der durch die Klammern 8 gebildeten Cliplinie. Ferner sollen die Rückhaltelaschen 35 so ausgebildet sein, wie Widerhaken, die in einer Richtung überfahrbar und verdrängbar durch die Klammern 8 sind.

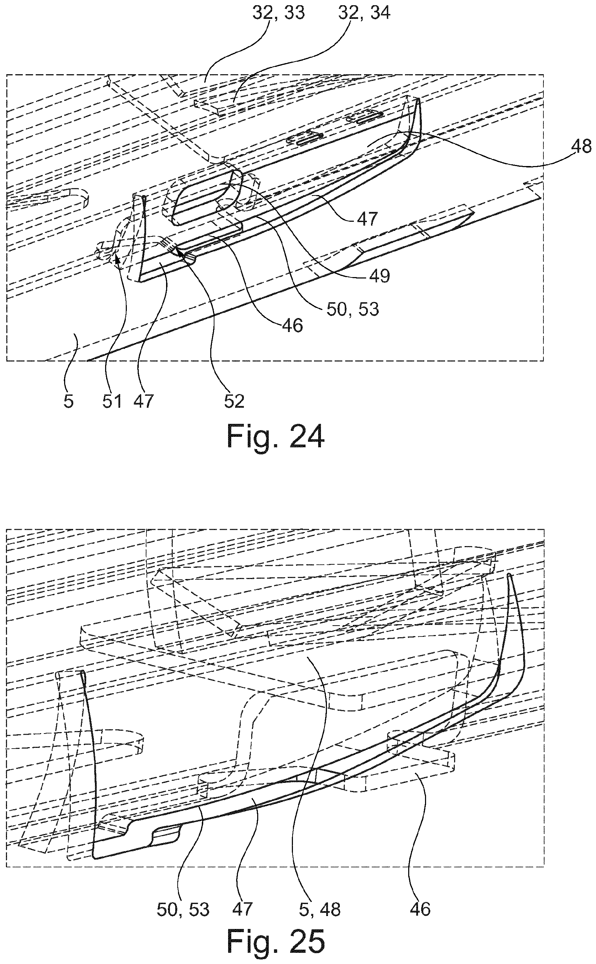

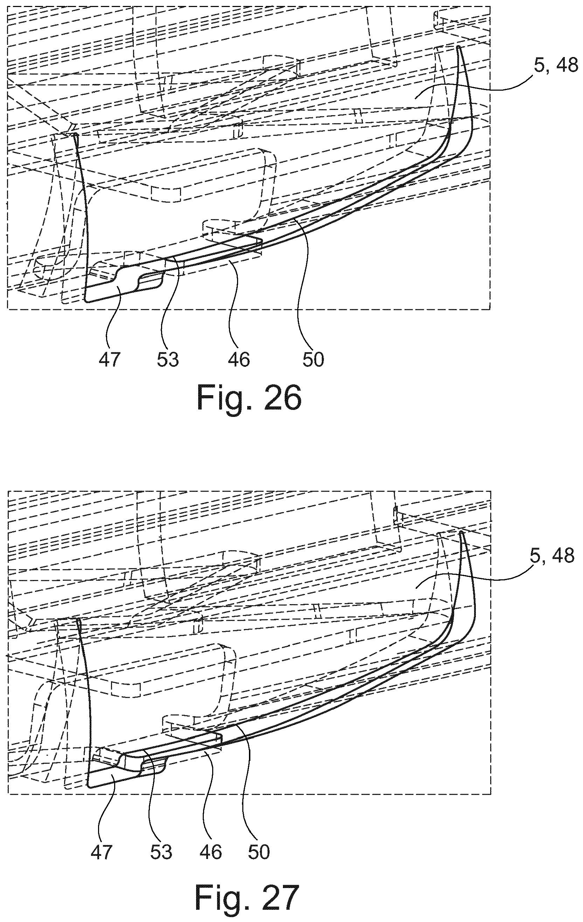

Die Rückhalteschiene 12 soll auch mittels Rückhalteschienen-Montagelaschen 46, wie sie in den Fig. 24 bis 27 erkennbar sind, an der Rohrwand des Außenrohres 5 fixiert werden. Dazu ist in dem Außenrohr 5 ein Montageloch 47 ausgebildet. Das Montageloch 47 ist bspw. mittels einer Laser-Schneidaktion schlitzartig eingebracht. Ein Außenrohr-Klappbereich 48, nach Art einer Lasche, mit einem Sichtfenster 49, ist radial nach innen gestellt und gibt genügend Platz zum Untergreifen der Rückhalteschienen-Montagelasche 46 unter eine durch den Außenrohr-Klappbereich 48 begrenzte Unterkante 50.

Dadurch ergibt sich ein axialer Anschlag nach proximal, versehen mit dem Bezugszeichen 51 und ein axialer Anschlag nach distal, versehen mit dem Bezugszeichen 52. Durch die Unterkante 50 wird eine Höhenfixierung 53 erreicht.

Das Montageloch 47 ist nach Art eines Fensters ausgestaltet. Das Sichtfenster 49 dient zur Kontrolle bei der Montage. Auf diese Weise wird ein selbstfangendes System gestaltet. Der Außenrohr-Klappbereich 48, der als Lasche agiert, und am Außenrohr 5 einstückig ausgebildet ist, fängt die Rückhalteschiene-Montagelasche 46, die ein einziger Bestandteil der als Rückhalteblech ausgebildeten Rückhalteschiene 12 ist und fixiert das Rückhalteblech in einer vorbestimmten Höhe und axialen Stellung.

Die Abfolge während der Montage geht aus den Fig. 25 bis 27 hervor. Es werden dort die Montagevorsprünge der Rückhalteschiene 12 von proximal nach distal unterfangen bzw. verfangen am Außenrohr-Klappbereich 48 mit der Rückhalteschiene-Montagelasche 46 dargestellt. In Fig. 24 ist der vollmontierte Zustand dargestellt.

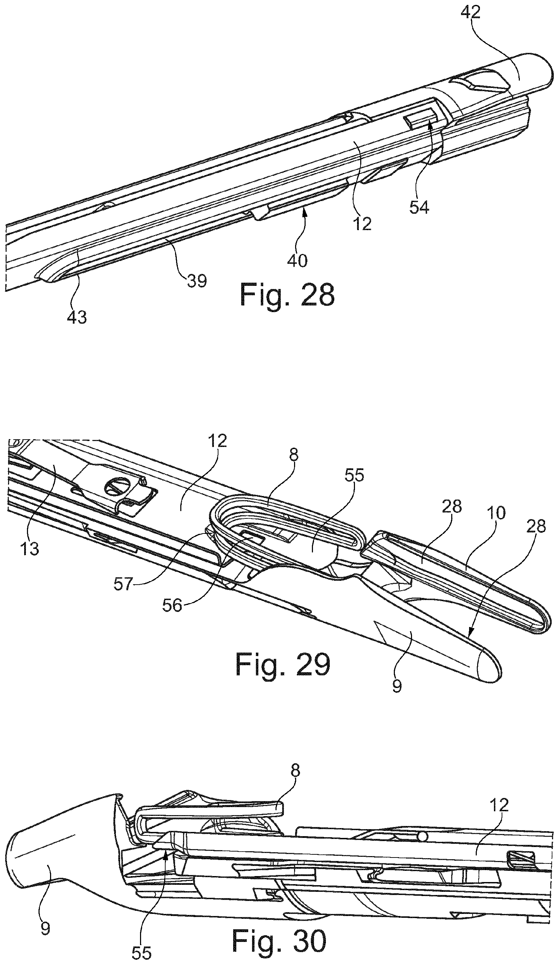

Ein dazu abgewandeltes Ausführungsbeispiel ist in der Fig. 28 gezeigt, bei der die Rückhalteschiene 12 mittels Ausschnitten am Federgegenhalter 42 fixiert ist. Der Federgegenhalter 42 ist nämlich seinerseits bereits am Außenrohr 5 radial und axial festgelegt. Dabei weist der Federgegenhalter 42 Nocken 54 auf, die durch die Rückhalteschiene 12 greifen, und beide Bauteile form- und/oder kraftschlüssig aneinander festlegen. Auch hier greift wieder ein Nocken 40 durch Langloch 39, so dass beim Anschlagen des Nockens 40 an der Anschlagkante 43 eine Förderhubbegrenzung erreicht wird, die von Vorteil bei einer kraftgesteuerten Handgriffverwendung ist. Ab einem bestimmten Grenzwert schaltet nämlich dann der Handgriff die Bewegungsrichtung um. Ein aktives Zurückholen der Förder- und Mitnahmeschiene 16 und der Zunge 13 ist dann realisierbar.

In den Fig. 29 bis 31 ist das Augenmerk auf ein distales Ende der Rückhalteschiene 12 gerichtet. Dort ist nämlich eine Brücke / Anlageplatte 55 ausgebildet, die das Gleiten der Klammer 8 in die Schalenformen 28 der oberen Maulteil-Branche 9 und der unteren Maulteil-Branche 10 erleichtert. Die Brücke / Anlageplatte 55 kann auch als Deckel zum unteren Maulteil 10 bezeichnet werden. Es gibt auch ein Rückhalteschienen-Montageloch 56, das zwischen der Brücke / Anlageplatte 55 und einem kicker- bzw. springschanzenartigen Abweiser 57 angeordnet ist. Dieser Abweiser 57 dient nach Art eines Kickers zum Anheben der Klammer 8 an ihrem Heck, d.h. an ihrem proximalen Ende, so dass die Klammer 8 besser in die obere und untere Maulteil-Branche 9 bzw. 10 eingleitet. Auch dient der Abweiser 57 einer Versteifung. Das Rückhalteschienen-Montageloch 56 wird von einem Montagewerkzeug (nicht gezeigt) während der Montage benutzt, um dort eingehängt zu werden.

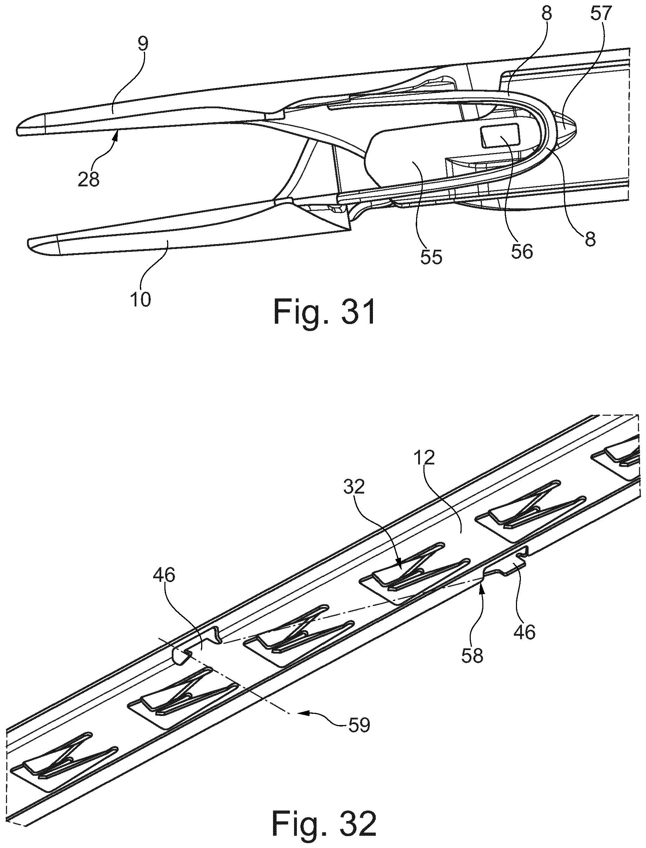

In der Fig. 32 sind in Längsrichtung versetzte, seitlich abstehende Rückhalteschienen-Montagelaschen 46 über eine (theoretische) asymmetrische Biegelinie 58 miteinander verbunden, so dass sich die mit dem Bezugszeichen 59 versehene (theoretische) direkte Biegelinie 59 nicht ergibt. Eine solche direkte Biegelinie 59, orthogonal zur Längsrichtung, wird vermieden, da die bei Auftreten von Torsionskräften kritische asymmetrische Biegelinien 58 bevorzugt wird, weil eben keine Torsionskräfte auftreten.

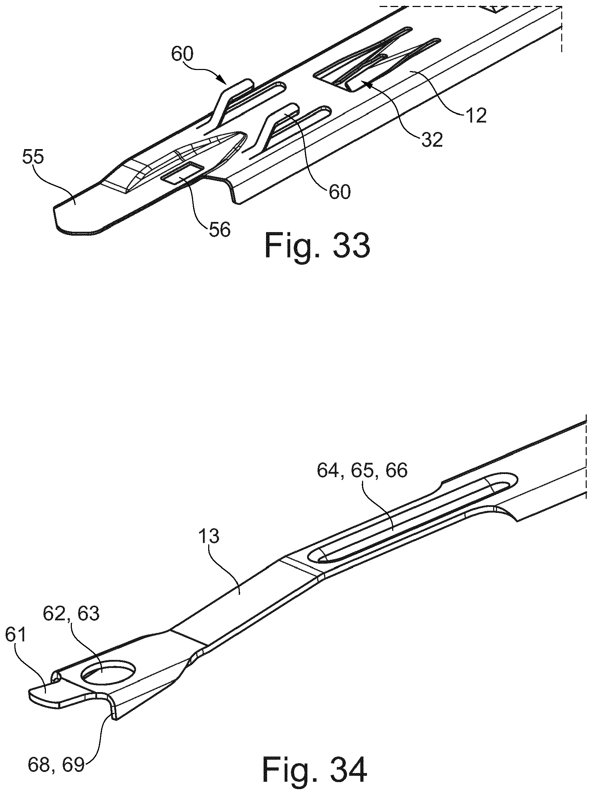

In der Fig. 33 sind Federlaschen 60 zu erkennen, die zum Abstützen der Rückhalteschiene 12 auf dem Schubrohr 21 dienen. Es wird also eine Restkraft zur Verfügung gestellt, die versucht, den Clipkanal, in den die Klammern 8 geleitet werden, zu verkleinern. Dies stabilisiert alle Bauteile zueinander und verhindert, dass die Klammern 8 beim Zurückziehen die Rückhaltelaschen 32 verpassen. Die sonst nötig werdenden höheren Rückhaltelaschen 32 würden dann mehr Kraft benötigen, was zu einer erhöhten Reibung führen würde, was dann einen vermehrten Kraftaufwand, während des Vorschubvorgangs nach sich ziehen würde. Letztlich wird auch ein Toleranzausgleich durch die Federlaschen realisiert. Die Federlaschen 60 können sich alternativ oder zusätzlich an der oberen und/oder unteren Maulteil-Branche 9 bzw. 10 abstützen.

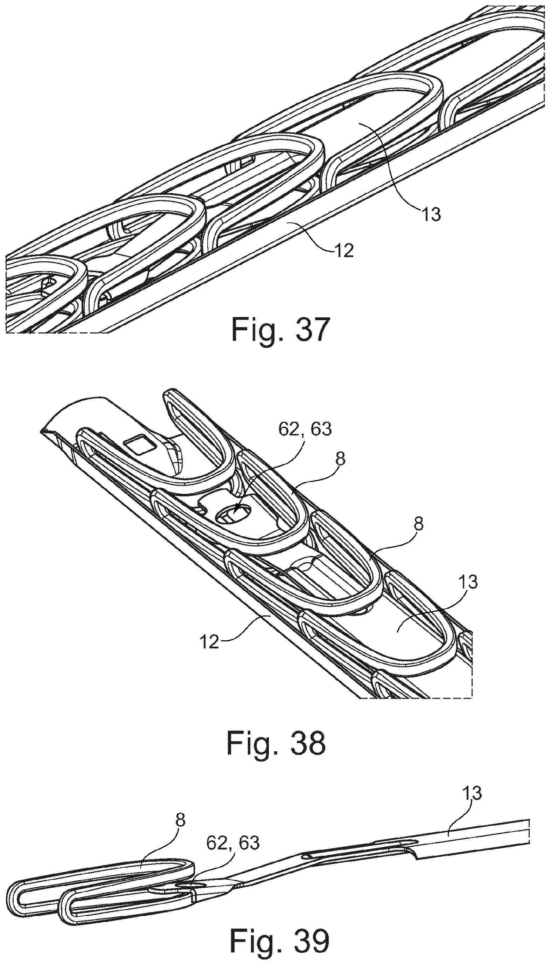

In den Fig. 34 bis 43 wird das Augenmerk nun auf die Zunge 13 und ihre spezielle Ausgestaltung gerichtet. So weist die Zunge 13 an ihrem distalen Ende einen Gewebeschutzüberstand 61 auf, der verhindert, dass Gewebe des zu behandelnden Organs zwischen die Klammerstege / Schenkel 29 der Klammer 8 gelangen kann und dort ungewollterweise eingeklemmt wird. Der Gewebeschutzüberstand 61 kann auch als Gewebeabstandshalter 61 bezeichnet werden. Proximal dazu ist ein Ausbruch 62 vorgesehen, der eine Eintauchöffnung 63 ausformt. Die Eintauchausnehmung kann auch synonym als Eintauchöffnung 63 bezeichnet werden.

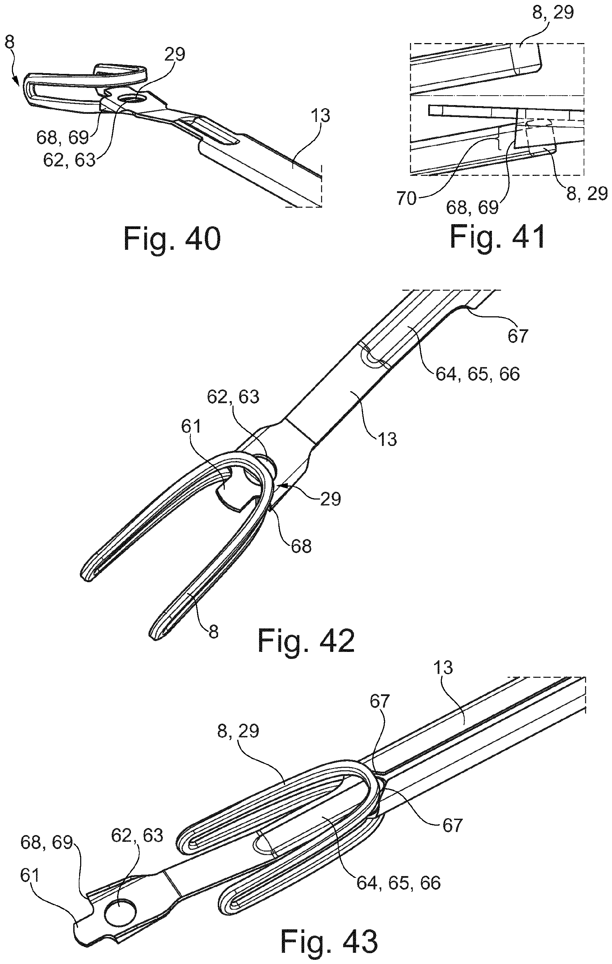

Wie besonders gut in Fig. 40 und 41 zu erkennen ist, ermöglicht diese Eintauchöffnung 63 das Einschwenken oder Eintauchen eines proximalen Abschnitts der Klammer 8, also eines Klammersteges / Schenkels 29, in die durch den Ausbruch 62 geschaffene Freihaltung. Die Eintauchöffnung 63 kann auch als Eintauchausnehmung bezeichnet werden. Sie ermöglicht, dass ein Abschnitt der Klammer 8 von unten zumindest ein Stück in den Ausbruch 62 hinein oder sogar hindurch ragt.

Auf der Unterseite der Zunge 13 ist auch ein Vorsprung ausgebildet, nach Art eines Bewegungsleitelementes 64. Dieses Bewegungsleitelement 64 ist nach Art eines Abweisers 65 oder einer Leitsicke 66 ausgebildet. Sie wirkt biegesteifigkeitserhöhend und gleichzeitig derart, dass ein Einhakschutz geschaffen wird. Der Einhakschutz verhindert, dass die Klammer 8 im Bereich von Stoßkanten 67 ungewünschterweise mit der Zunge 12 in Kontakt gelangt, da sonst die Klammer 8 zu früh oder falsch in Axialrichtung bewegt würde. Wohl sind auch Anlagekanten 68 vorgehalten, um gezielt die Klammer 8 zu schieben. Diese Anlagekante 68 kann auch als Schiebekante oder Drucklasche bezeichnet werden. Sie ist deshalb auch mit dem Bezugszeichen 69 versehen. Über die mit der Bezugslinie 70 bezeichnete Länge liegt dann die Zunge an einem Teil eines Klammersteges 29 an. Die Eintauchöffnung 63 ermöglicht in dem Zusammenhang auch, dass die Anlage zwischen der Zunge 13 und der Klammer 8 beim Abkippen der Klammer 8 erhalten bleibt.

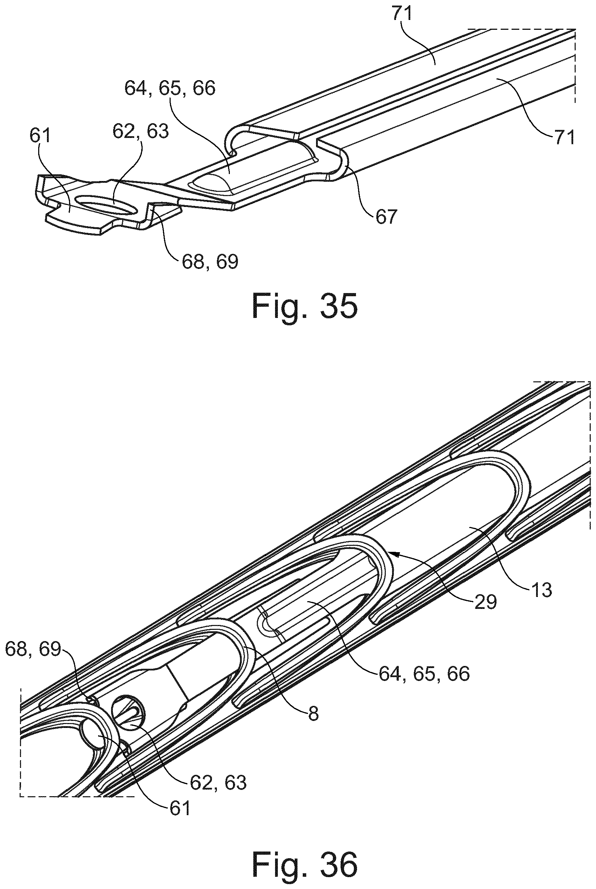

Der Gewebeschutzüberstand 61, wie er schützend auf tierisches oder menschliches Gewebe wirken kann, ist gut aus Fig. 34 deduzierbar. Die Leitwirkung des Bewegungsleitelements 64 / Abweisers 65 / der Leitsicke 66 ist gut der Fig. 35 bis 39 und 43 entnehmbar. Dort wird auch klar, dass die Leitsicke 66 vermeidet, dass sich die Klammer 8 verhakt. Das Bewegungsleitelement 64 ist auch steifigkeitserhöhend für die Zunge 13. Somit werden ein Einhakschutz und eine Kantenabschirmung zusätzlich zu einer Ausknickvermeidung realisiert. Die Klammer 8 kann sich daher nicht an einem Blechumschlag der Zunge 13 einhaken. Dies kann auch als "Kanten-Shielding" bezeichnet werden.

Die Zunge 13 wird durch mehrere Klammern 8 hindurchgefädelt, ohne eine Führung derselben zu übernehmen. Sie ist ein langgestrecktes Element mit hoher Knickneigung zum Vorschieben und Führen des am weitesten distal gelegensten Clips / der am weitesten distal gelegenen Klammer 8, mit geringster Toleranz. Durch eine längsseitige Bördelung 71 wird ein Ausknicken der Zunge 13 verhindert. Auch wirken Längssicken, wie eine in den Fig. 34 bis 43 gezeigt ist, knickverhindernd. An der Zungenspitze der Zunge 13 wird ein sicheres Greifen der Klammer 8 erreicht, genauso wie ein Übertragen von Kräften auf die Klammer 8 bei einem Winkel- und einem Lagewechsel, ebenso wie das Abfangen von Toleranzen. Durch das Ausgestalten der Spitze als flexiblen Abschnitt, etwa durch dünneres Material, durch das Vorhalten von Elastizitätslöchern, durch Ausschnitte zur Verringerung der Biegekräfte, wird eine vorteilhafte Ausführungsform realisierbar. Vorteilhaft sind spezielle Laschen- und Spitzenformen, die ein Aushebein der Drucklaschen 69 verhindern. Ein Abrutschen der Zunge 13 von einem Klammersteg / Schenkel 29 wird in der weiter oben beschriebenen Weise wirkungsvoll verhindert.

Der Gewebeschutzüberstand 61 überragt das Blech an der Zungenspitze und ist in Anlage auf der Innenseite einer Klammer 8 in der dort ausgebildeten Schenkelkehle. Es sei noch angemerkt, dass der Abweiser 65 / die Leitsicke 66 eine solche Tiefe aufweist, wie die Bördelung 71 der Zunge 13, um eine Schubweitergabe an die Klammer 8 zu verhindern.

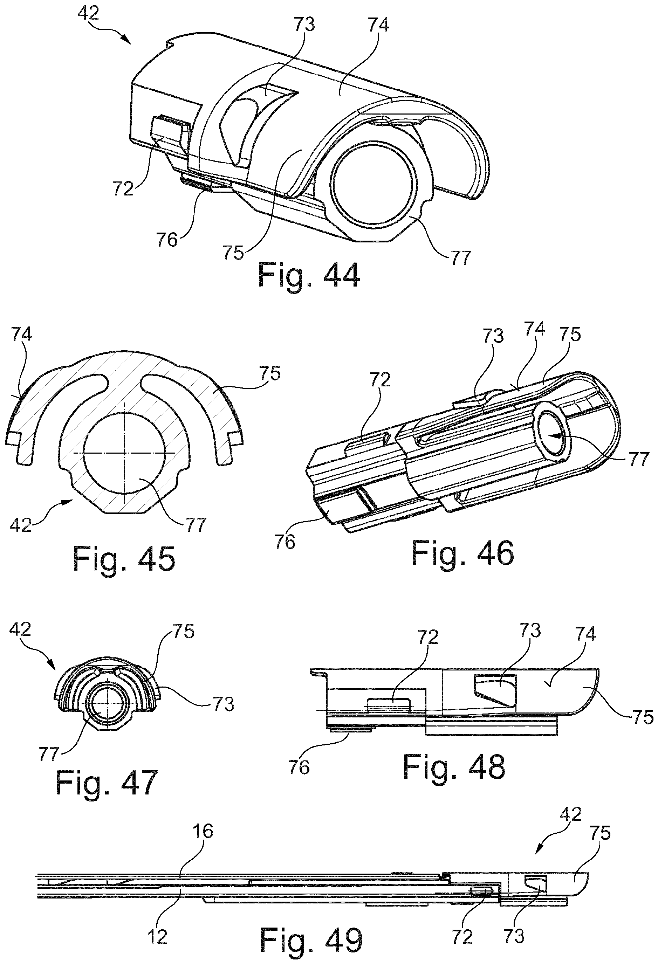

In den nachfolgenden Fig. 44 bis 51 wird der Federgegenhalter 42 näher dargestellt und erläutert. So weist der Federgegenhalter 42 eine Halteraste / einen Nocken 72 auf, der zum Einhaken in die Rückhalteschiene 12 vorgesehen ist, um eine Axial- und/oder Radialfixierung vorzunehmen. Ferner weist der Federgegenhalter 42 einen Nocken / eine Lasche 73 auf zum Festlegen des Federhalters an dem Außenrohr 8.

Dieser Nocken 73 ist von einer Dichtfläche 74 umgeben. Sie dient dazu, die Ausnehmung im Außenrohr 5 abzudichten, so dass kein Fluid aus dem Schaftinstrument nach außen gelangen kann, genauso wenig wie Fluide von außerhalb des Schaftinstruments 1 in dessen Inneres gelangen soll. Die die Dichtfläche 74 ausbildende Gegend, ist nach Art einer Domfeder ausgestaltet, welche mit dem Bezugszeichen 75 versehen ist. Der keilförmige Nocken 73 liegt somit innerhalb einer Dichtfläche 74 der Domfeder 75. Diese Domfeder 75 verstemmt sich radial in eine Richtung, weil sie nicht konzentrisch angeordnet ist.

Die Halteraste 72 und eine diese aufnehmende Ausnehmung kann so ausgebildet sein, dass ein Festlegen des Rückhalteblechs 12 am Federgegenhalter 42 in Radialrichtung und Axialrichtung getrennt voneinander stattfindet. Die Toleranzen können dann sinnvoller genutzt werden, als wenn dies an einem Bauteil in beide Richtungen geschehen würde. Deshalb ist auch ein Vorsprung 76 vorgesehen, der lediglich zum axialen Halten der Rückhalteschiene 12

20

ausgelegt ist, wohingegen die Halterasten 72 zum radialen Fixieren ausgelegt sind. Der Federgegenhalter 42 hat somit eine Shell-Form, welche positiv für die Elastizität ist. Der Federgegenhalter 42 dient der Fixierung am Außenrohr 5. Er kann als Spritzgussteil mit einer zentralen Durchführung zur Führung der Schubstange / Vorschubstange 19 ausgebildet sein. Er kann nach Art einer integrierten Ringfeder ausgebildet sein und ein federndes, dünnwandiges Übermaßgewölbe aufweisen. Eine flächige Anlage um den Nocken 73, der nach Art einer Haltenocke ausgestaltet ist, herum, um eine Dichtigkeit zu erzeugen, ist von Vorteil. Ein zentrierender Vorsprung / Haltenocken greift einfacher ins Außenrohr 5 ein, wenn er Abschrägungen aufweist. Solche Abschrägungen können bei allen Vorsprüngen oder Laschen vorhanden sein. Der Federgegenhalter 42 clipst sich an der Rückhalteschiene 12 fest. Der Federgegenhalter 42 weist somit ein Durchgangsloch 77 in Längsrichtung auf, das die Führung der Vorschubstange 19 übernimmt.

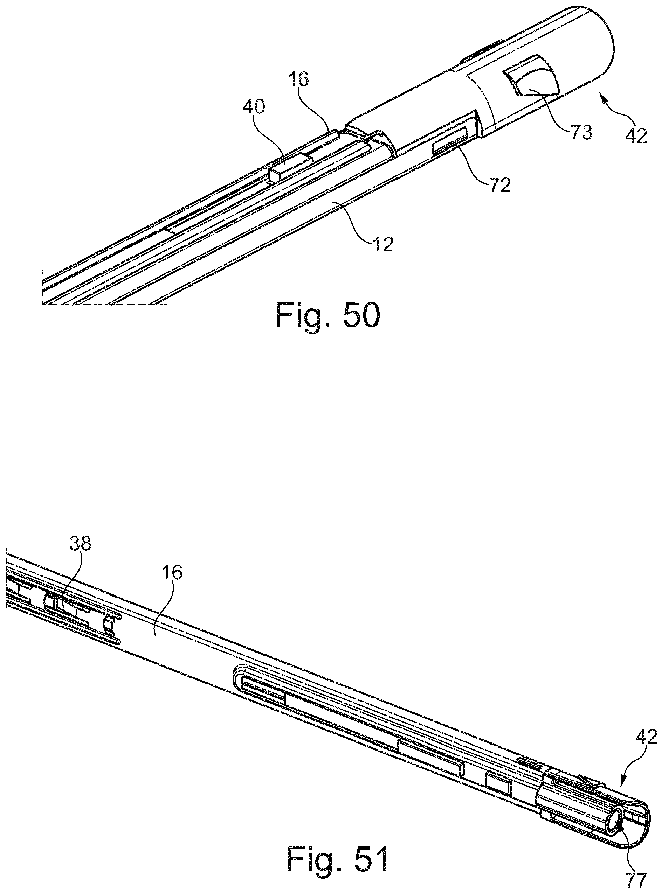

Die Fig. 50 zeigt den Zustand des Federhalters 42 im eingeclipsten Zustand in das Rückhalteblech, welches die Rückhalteschiene 12 ausformt. Diese Situation ist auch in Fig. 51 dargestellt.

Die Brücke / Anlageplatte 55 kann auch als Auflageplatte bezeichnet werden. Das Rückhalteschienen-Montageloch 56 kann auch verkürzt als Montageloch bezeichnet werden.

Bezuqszeichenliste

| | |
|---|---|
| 1 | medizinisches Schaftinstrument |
| 2 | Instrumentengriff |
| 3 | Instrumentenkopf |
| 4 | Instrumentenschaft |
| 5 | Außenrohr |
| 6 | Gehäuse |
| 7 | Clipmagazin |
| 8 | Klammer |
| 9 | oberes Maulteil / obere Maulteil-Branche |
| 10 | unteres Maulteil / untere Maulteil-Branche |
| 11 | Schieber / Nockenträgerbauteil |
| 12 | Rückhalteschiene |
| 13 | Zunge / Vorschubzunge |
| 14 | Vorschubaufteiler |
| 15 | Federgegenhalter |
| 16 | Vorschubschiene / Förder- und Mitnahmeschiene |
| 17 | Vorschubfeder |
| 18 | Dichtscheibe |
| 19 | Vorschubstange |
| 20 | Vorschubstangenendstück |
| 21 | Schubrohr |
| 22 | Dichtring |
| 23 | Druckfeder |
| 24 | Federflansch |
| 25 | Griffflansch |
| 26 | Griffstück |
| 27 | Hohlzylinderendstück |
| 28 | Schalenendform |
| 29 | Klammersteg / Schenkel |
| 30 | Berührungsfläche / Anlagefläche |
| 31 | Innenwandung / Innenseite |
| 32 | Rückhaltelasche |
| 33 | erster Rückhaltelaschenabschnitt |
| 34 | zweiter Rückhaltelaschenabschnitt |
| 35 | Schwenk- / Biegelinie |
| 36 | distale Kante der Rückhaltelasche |
| 37 | Seitenkante der Rückhaltelasche |
| 38 | Klammer-Mitnahmeelement / -lasche |
| 39 | Langloch |
| 40 | Nocken für Vorschubaufteiler |

41    Vorschubaufteiler
42    Federgegenhalter
43    Anschlagkante
44    Ausnehmung
45    Falzblech
46    Rückhalteschienen-Montagelasche
47    Montageloch in Außenrohr
48    Außenrohr-Klappbereich
49    Sichtfenster
50    Unterkante des Außenrohr-Klappbereiches
51    axialer Anschlag nach proximal
52    axialer Anschlag nach distal
53    Höhenfixierung
54    Federgegenhaltenocken
55    Brücke / Anlageplatte
56    Rückhalteschiene-Montageloch
57    Abweiser
58    asymmetrische Biegelinie
59    direkte Biegelinie
60    Federlasche
61    Gewebeschutzüberstand
62    Ausbucht
63    Eintauchöffnung
64    Bewegungsleitelement
65    Abweiser
66    Leitsicke
67    Stoßkante
68    Anlagekante
69    Schiebekante / Drucklasche
70    Anlagelänge
71    Bördelung
72    Halteraste / Nocken des Federgegenhalters für Rückhalteschiene
73    Nocken / Lasche des Federgegenhalters für Außenrohr
74    Dichtfläche
75    Domfeder
76    Vorsprung des Federgegenhalters für Rückhalteschiene
77    Durchgangsloch

**Patentansprüche**

1.  Medizinisches Schaftinstrument (1), mit einem Instrumentenkopf (3) zum Applizieren von Klammern (8), wobei der Instrumentenkopf (3) über einen Instrumentenschaft (4) mit einem Instrumentengriff (2) zur Betätigung des Instrumentenkopfes (3) verbindbar ist, und mit einem ein Gehäuse (6) aufweisenden Clipmagazin (7), in welchem eine Vielzahl der Klammern (8) nach einem Einlagerungsprinzip mit einem vorbestimmten Lagerpositionsabstand zueinander gebunkert sind, welche sämtlich mittels einer hin- und herbewegbaren Förder- und Mitnahmeschiene (16) des medizinischen Schaftinstruments (1) im Rahmen eines einzigen Förderhubs um jeweils eine Lagerposition vorrückbar sind, wobei die Förder- und Mitnahmeschiene (16) pro Klammer (8) ein Klammer-Mitnahmeelement (38) aufweist,

    wobei der Relativabstand der einzelnen Klammer-Mitnahmeelemente (38) unterschiedlich zu den jeweiligen Lagerpositionsabständen der Klammern (8) ist, derart, dass die Mitnahme der gebunkerten Klammern (8) im Rahmen eines einzigen Förderhubes getaktet erfolgt,
    wobei das medizinische Schaftinstrument (1) eine Rückhalteschiene (12) mit Rückhaltelaschen (32) aufweist,
    **dadurch gekennzeichnet, dass**
    die Abstände zwischen den Klammer-Mitnahmeelementen (38) unterschiedlich sind,
    wobei die Abstände zwischen den Rückhaltelaschen (32) unterschiedlich sind, wobei die relative Veränderung der Abstände zwischen den Klammer-Mitnahmeelementen (38) einerseits und den Rückhaltelaschen (32) andererseits kleiner oder größer ist.

2. Medizinisches Schaftinstrument (1) nach Anspruch 1, **dadurch gekennzeichnet, dass** jene die Klammern (8) fördernden und führenden Einrichtungen und die Klammern (8) so aufeinander abgestimmt sind, dass die Klammern (8) in anderen Abständen zwischen zwei Förderhüben im Schaftinstrument (1) abgelegt werden, als durch die fördernden Einrichtungen vorgegeben ist.

3. Medizinisches Schaftinstrument (1) nach Anspruch 1 oder 2, **dadurch gekennzeichnet, dass** die Klammer-Mitnahmeelemente (38) die Lagerpositionen, die von den Rückhaltelaschen (32) der Rückhalteschiene (12) vorbestimmt sind, die Klammern (8) und die die Klammern (8) bei ihrer Bewegung nach distal bewegende Förder- und Mitnahmeschienen (16) so aufeinander abgestimmt sind, dass als erstes eine proximale Klammer (8) und zeitlich nachfolgend während des Förderhubs die distal vorgelagerten Klammern (8) bewegt werden oder als erstes eine distale Klammer (8) und zeitlich nachfolgend die proximal nachgelagerten Klammern (8) bewegt werden.

4. Medizinisches Schaftinstrument (1) nach einem der vorstehenden Ansprüche, **dadurch gekennzeichnet, dass** die Rückhaltelasche (32) beim Überfahrenwerden von den Klammern (8) bei deren Weg zum distalen Ende des Instrumentenkopfes (3) aus dem Bewegungspfad der Klammern (8) ausreichend biegbar oder verschwenkbar an der Rückhalteschiene (12) angebunden sind, wobei es beim Rückzug des Vorschubelements es die Förderelemente sind die ausweichen.

5. Medizinisches Schaftinstrument (1) nach Anspruch 4, **dadurch gekennzeichnet, dass** die Rückhaltelasche (32) nach Art von einer Schmetterlingslasche (33, 34) um eine quer zur Längsrichtung der Rückhalteschiene (12) ausgerichtete Biege- oder Schwenkachse biegbar oder schwenkbar angebunden ist.

6. Medizinisches Schaftinstrument (1) nach Anspruch 5, **dadurch gekennzeichnet, dass** zwei Flügel einer Schmetterlingslasche (33, 34) eine Rückhaltelasche (32) ausbilden, wobei jeder Flügel um eine Biege- oder Schwenklinie (35) biegbar oder schwenkbar ist, die sich distal oder proximal der Rückhaltelasche (32) kreuzen.

7. Medizinisches Schaftinstrument (1) nach einem der vorstehenden Ansprüche, **dadurch gekennzeichnet, dass** die Rückhaltelaschen (32) geometrisch und stofflich so ausgestaltet sind, dass sie sich beim Überfahrenwerden durch die Klammern (8) näherungsweise flach legen.

8. Medizinisches Schaftinstrument (1) nach einem der vorstehenden Ansprüche, **dadurch gekennzeichnet, dass** die quer zur Axialrichtung gemessene Höhe zumindest einiger der Rückhaltelaschen (32) so groß ist, dass sie eine durch die Klammern (8) hindurchgeführte Zunge (13) zum Ausstoßen der distalsten Klammer (8) führen und/oder ein Ausknicken der Zunge (13) verhindert.

9. Medizinisches Schaftinstrument (1) nach einem der vorstehenden Ansprüche, **dadurch gekennzeichnet, dass** die durchschnittliche Abstandsänderung des die Klammern kontaktierenden Formschlusselementen zum durchschnittlichen Abstand etwa 0,1% bis 4% beträgt. Vorzugsweise jedoch 0,8% bis 1,5% beträgt, wobei die Formschlusselemente ein Teil der Förder- und Mitnahmeschiene oder der Rückhalteschiene sind.

10. Medizinisches Schaftinstrument (1) nach einem der vorstehenden Ansprüche, **dadurch gekennzeichnet, dass** die Rückhalteschiene (12) als Rückhalteblech aus einem metallischen Werkstoff ausgebildet ist.

11. Medizinisches Schaftinstrument (1) nach einem der vorhergehenden Ansprüche, mit dem ein Maulteil (9, 10) aufweisenden Instrumentenkopf (3) zum Applizieren von Klammern (8) der über den ein Außenrohr (5) aufweisenden Instrumentenschaft (4) mit dem Instrumentengriff (2) zur Betätigung des Instrumentenkopfes (3) verbindbar ist und dem Clipmagazin (7), in welchem die Rückhalteschiene (12) zur Auflagerung von Klammern (8) nach dem Einlagerungsprinzip mit einem vorbestimmten Lagerpositionsabstand zueinander fixiert ist, wobei die Klammern (8) sämtlich mittels der hin- und herbewegbaren Förder- und Mitnahmeschiene (16) im Rahmen eines einzigen Förderhubs um jeweils eine Lagerposition verrückbar sind, wofür an der Rückhalteschiene (12) und an der Förder- und Mitnahmeschiene (16) voneinander beabstandete Rückhaltelaschen (32) einerseits und Klammer-Mitnahmeelemente (38) andererseits vorgesehen sind, welche beim Übergleiten der Klammern (8) in die jeweils andere Richtung federelastisch aus der Kammergleitbahn wegschwenkend ausgestaltet sind, **dadurch gekennzeichnet, dass** eine Anzahl der Rückhaltelaschen (32) und/oder Klammer-Mitnahmeelemente (38) in einer Schmetterlingsform ausgebildet sind, mit zwei in Förderrichtung zueinander abkippbaren Anschlagsflügel ausgebildet sind, und/oder eine Anzahl der Rückhaltelaschen (32) und/oder Klammer-Mitnahmeelemente (38) in einer Stützkeilform ausgebildet sind, mit einer quer zur Förderrichtung verschwenkbaren Kuppe, an deren distalen freien Kante.

**12.** Medizinisches Schaftinstrument (1) nach Anspruch 11, **dadurch gekennzeichnet, dass** eine im Wesentlichen senkrecht oder zumindest schräg zur Förderrichtung verlaufende Anschlagsplatte oder Anschlagsfläche an den Rückhaltelaschen (32) und/oder Klammer-Mitnahmeelementen (38) ausgebildet ist / sind.

**13.** Medizinisches Schaftinstrument (1) nach einem der Ansprüche 11 oder 12, **dadurch gekennzeichnet, dass** eine Zunge (13) durch die Förder- und Mitnahmeschiene (16) und/oder die Rückhalteschiene (12) abgestützt ist.

**14.** Medizinisches Schaftinstrument (1) nach Anspruch 13, **dadurch gekennzeichnet, dass** von der Rückhalteschiene (12) in Richtung der Zunge (13) diese unterstützende Federlaschen (60) abstehen.

**Claims**

**1.** Medical shaft instrument (1), with an instrument head (3) for applying clamps (8), the instrument head (3) being connectable via an instrument shaft (4) to an instrument handle (2) for actuating the instrument head (3), and a clip magazine (7) having a housing (6), in which magazine a plurality of clamps (8) are bunkered at a predetermined storage position spacing relative to each other according to a storage principle, all the clamps being movable forwards, each by one storage position by a reciprocatable transport and entraining rail (16) of the medical shaft instrument (1) in one single delivery stroke, the transport and entraining rail (16) having one clamp entraining element (38) per clamp (8),
the relative distance between the individual clamp entraining elements (38) differing from the respective storage position distances of the clamps (8) in such a way that the entrainment of the bunkered clamps (8) is carried out in one single delivery stroke in a clocked way, the medical shaft instrument (1) having a retaining rail (12) with retaining lugs (32),
**characterised in that**
the distances between the clamp entraining elements (38) are different,
wherein the distances between the retaining lugs (32) are different,
wherein the relative change in the distances between the clamp entraining elements (38) on the one hand and the retaining lugs (32) on the other hand are smaller or larger.

**2.** Medical shaft instrument (1) according to claim 1, **characterised in that** said apparatuses for transporting and guiding the clamps (8), and said clamps (8), are adapted to one another such that the clamps (8) are deposited between two delivery strokes in the shaft instrument (1) in other distances than predefined by the transporting apparatuses.

**3.** Medical shaft instrument (1) according to claim 1 or 2, **characterised in that** the clamp entraining elements (38), storage positions predetermined by the retaining lugs (32) of the retaining rail (12), the clamps (8) and the transport and entraining rails (16) moving the clamps (8) during movement in a distal direction are adapted to one another such that initially, a proximal clamp (8) is moved, and subsequently during the delivery stroke, the distally downstream clamps (8) are moved, or initially, a distal clamp (8) is moved, and subsequently proximally upstream clamps (8) are moved.

**4.** Medical shaft instrument (1) according to any of the preceding claims, **characterised in that** the retaining lug (32), when run over by the clamps (8) on their way to the distal end of the instrument head (3), is bound on the retaining rail (12) in a bendable or swivelling way out of the motion path of the clamps (8) to a sufficient extent, wherein during the retraction of the advancing element the transport elements get out of the way.

**5.** Medical shaft instrument (1) according to claim 4, **characterised in that** the retaining lug (32), in the manner of a butterfly lug (33, 34), is bound in a bendable or swivelling way about a bending or swivel axis which is aligned transverse to a longitudinal direction of the retaining rail (12).

**6.** Medical shaft instrument (1) according to claim 5, **characterised in that** two wings of a butterfly lug (33, 34) form a retaining lug (32), with each of the wings being bendable or swivelling about a bending or swivel line (35), crossing one another distally or proximally relative to the retaining lug (32).

**7.** Medical shaft instrument (1) according to any of the preceding claims, **characterised in that** the geometry and the material of the retaining lugs (32) are configured such that said retaining lugs lie down substantially flat when run over by the clamps (8).

8. Medical shaft instrument (1) according to any of the preceding claims, **characterised in that** the height of at least some of the retaining lugs (32) measured transverse to the axial direction is so large that it guides a tongue (13), which is guided through the clamps (8) for ejecting the most distal clamp (8) and/or **in that** it prevents a buckling of the tongue (13).

9. Medical shaft instrument (1) according to any of the preceding claims, **characterised in that** the average spacing alteration of the form-fitting elements contacting the clamps in relation to the average spacing is approximately 0.1% to 4%. But preferably 0.8% to 1.5%, the form-fitting elements being a part of the transport and entraining rail or of the retaining rail.

10. Medical shaft instrument (1) according to any of the preceding claims, **characterised in that** the retaining rail (12) as a retaining plate is made from a metallic material.

11. Medical shaft instrument (1) according to any of the preceding claims, with the instrument head (3) having a jaw part (9, 10) and intended for applying clamps (8), the jaw part being connectable to an instrument handle (2) for actuating the instrument head (3) via the instrument shaft (4) comprising an outer tube (5), and the clip magazine (7), in which the retaining rail (12) is fixed for storing clamps (8) at a predetermined storage position spacing relative to each other according to the storage principle, wherein all the clamps (8) are movable forwards, each by one storage position by the reciprocatable transport and entraining rail (16) in one single delivery stroke, for which purpose retaining lugs (32) spaced apart from each other, on the one hand, and clamp entraining elements (38), on the other hand, are provided on the retaining rail (12) and on the transport and entraining rail (16), which retaining lugs (32) and clamp entraining elements (38), when the clamps (8) slide over them, are designed so as to swivel away out of the clamp slipway in the respectively other direction in an elastic fashion, **characterised in that** a number of the retaining lugs (32) and/or clamp entraining elements (38) are formed in a butterfly shape comprising two stop wings capable of being tilted relative to each other in the transport direction, and/or a number of the retaining lugs (32) and/or clamp entraining elements (38) are formed so as to have a supporting wedge shape comprising a tip which is pivotable transverse to the transport direction and is provided at a distal free edge.

12. Medical shaft instrument (1) according to claim 11, **characterised in that** a stop plate or stop face is/are formed on the retaining lugs (32) and/or clamp entraining elements (38) so as to extend substantially perpendicular or at least oblique to the transport direction.

13. Medical shaft instrument (1) according to claim 11 or 12, **characterised in that** a tongue (13) is supported by the transport and entraining rail (16) and/or the retaining rail (12).

14. Medical shaft instrument (1) according to claim 13, **characterised in that** spring lugs (60) project from the retaining rail (12) towards the tongue (13) and support said tongue.

**Revendications**

1. Instrument médical à tige (1) comportant une tête d'instrument (3) pour appliquer des agrafes (8), dans lequel la tête d'instrument (3) peut être reliée par l'intermédiaire d'une tige d'instrument (4) à une poignée d'instrument (2) pour actionner la tête d'instrument (3), et un chargeur d'agrafes (7) présentant un logement (6), dans lequel une pluralité d'agrafes (8) sont stockées selon un principe de stockage avec une distance prédéterminée entre les positions de stockage les unes par rapport aux autres, qui peuvent être avancées en totalité au moyen d'un rail de transport et d'entraînement (16) mobile en va en vient de l'instrument médical à tige (1) dans le cadre d'une course de transport unique jusque dans une position de stockage, dans lequel le rail de transport et d'entraînement (16) présente un élément d'entraînement d'agrafe (38) par agrafe (8),
dans lequel la distance relative des éléments d'entraînement d'agrafe (38) individuels est différente des distances entre les positions de stockage respectives des agrafes (8), de telle sorte que l'entraînement des agrafes stockées (8) est synchronisé dans le cadre d'une course de transport unique,
dans lequel l'instrument médical à tige (1) présente un rail de retenue (12) avec des pattes de retenue (32),
**caractérisé en ce que**
les distances entre les éléments d'entraînement d'agrafe (38) sont différentes,
dans lequel les distances entre les pattes de retenue (32) sont différentes,
dans lequel la modification relative des distances entre les éléments d'entraînement d'agrafe (38) d'une part et les pattes de retenue (32) d'autre part est inférieure ou supérieure.

**2.** Instrument médical à tige (1) selon la revendication 1, **caractérisé en ce que** les dispositifs transportant et guidant les agrafes (8) et les agrafes (8) sont mutuellement coordonnés les uns aux autres de sorte que les agrafes (8) sont placées à d'autres distances entre deux courses de transport dans l'instrument à tige (1) que celle prévue par les dispositifs de transport.

**3.** Instrument médical à tige (1) selon la revendication 1 ou 2, **caractérisé en ce que** les éléments d'entraînement d'agrafe (38) sont mutuellement coordonnées aux positions de stockage des agrafes (8), qui sont prédéterminées par les pattes de retenue (32) du rail de retenue (12), et aux rails de transport et d'entraînement (16) pouvant déplacer les agrafes (8) distalement par l'intermédiaire de leur déplacement, de sorte que tout d'abord une agrafe proximale (8) puis à la suite les agrafes en amont distales (8) sont déplacées pendant la course de transport, ou tout d'abord une agrafe distale (8) puis à la suite les agrafes en en aval proximales (8) sont déplacées.

**4.** Instrument médical à tige (1) selon l'une quelconque des revendications précédentes, **caractérisé en ce que** les pattes de retenue (32) sont reliées au rail de retenue (12) de manière suffisamment souple ou pouvant pivoter lors du passage des agrafes (8) sur leur chemin vers l'extrémité distale de la tête d'instrument (3) à partir du trajet de déplacement des agrafes (8), dans lequel lors d'un recul de l'élément d'avance, les éléments de transport sont esquivés.

**5.** Instrument médicale à tige (1) selon la revendication 4, **caractérisé en ce que** la patte de retenue (32), à la manière d'une patte de papillon (33, 34), est reliée pouvant fléchir ou pivoter à un axe de flexion ou de pivotement orienté transversalement par rapport à la direction longitudinale du rail de retenue (12).

**6.** Instrument médical à tige (1) selon la revendication 5, **caractérisé en ce que** deux ailes d'une patte de papillon (33, 34) forment une patte de retenue (32), dans lequel chaque aile peut être fléchie ou pivotée autour d'une ligne de flexion ou de pivotement (35), qui croisent de manière distale ou proximale la patte de retenue (32).

**7.** Instrument médical à tige (1) selon l'une quelconque des revendications précédentes, **caractérisé en ce que** les pattes de retenue (32) sont conçues géométriquement et matériellement de manière à être approximativement à plat lors du passage des agrafes (8).

**8.** Instrument médical à tige (1) selon l'une quelconque des revendications précédentes, **caractérisé en ce que** la hauteur mesurée transversalement à la direction axiale d'au moins une des pattes de retenue (32) est si grande qu'elle guide une languette (13) qui passe à travers les agrafes (8) pour éjecter l'agrafe la plus distale (8) et/ou empêche le flambage de la languette (13).

**9.** Instrument médical à tige (1) selon l'une quelconque des revendications précédentes, **caractérisé en ce que** la modification de distance moyenne des éléments de fermeture par liaison de forme venant en contact avec les agrafes correspond à une distance moyenne d'environ 0,1 % à 4 %. De préférence de 0,8 % à 1,5 %, dans lequel les éléments de fermeture par liaison de forme sont une partie du rail de transport et d'entraînement ou du rail de retenue.

**10.** Instrument médical à tige (1) selon l'une des revendications précédentes, **caractérisé en ce que** le rail de retenue (12) est conçu comme une tôle de retenue en un matériau métallique.

**11.** Instrument médical à tige (1) selon l'une des revendications précédentes, avec une tête d'instrument (3) présentant une partie de mâchoire (9, 10) qui peut être reliée à la poignée d'instrument (2) pour actionner la tête d'instrument (3) et au chargeur d'agrafes (7), afin d'appliquer des agrafes (8) de la tige d'instrument (4) présentant un tube extérieur (5), où le rail de retenue (12) destiné à supporter des agrafes (8) est fixé selon le principe de stockage avec une distance prédéterminée entre les positions de stockage les unes par rapport aux autres, dans lequel les agrafes (8) peuvent être décalées en totalité au moyen du rail de transport et d'entraînement (16) mobile en va en vient dans le cadre d'une course de transport unique jusque dans une position de stockage, auquel cas des pattes de retenue (32) espacées les unes des autres sont prévues sur le rail de retenue (12) et sur le rail de transport et d'entraînement (16), d'une part, et des éléments d'entraînement d'agrafe (38), d'autre part, sont prévus, qui sont conçu pouvant pivoter au loin par déformation élastique lors du glissement des agrafes (8) dans la glissière d'agrafe, **caractérisé en ce qu'**une pluralité des pattes de retenue (32) et/ou des éléments d'entraînement d'agrafe (38) sont conçus en forme de papillon, sont conçus avec deux ailes de butée qui peuvent être inclinées l'une vers l'autre dans la direction de transport et/ou un pluralité des pattes de retenue (32) et/ou des éléments d'entraînement d'agrafe (38) sont conçus en forme de coin de support, avec un ménisque pouvant pivoter transversalement à la direction de transport au niveau de son bord libre distal.

**12.** Instrument médical à tige (1) selon la revendication 11, **caractérisé en ce qu'**une plaque de butée ou une surface de butée s'étendant sensiblement perpendiculairement ou au moins obliquement par rapport à la direction de transport sur les pattes de retenue (32) et/ou des éléments d'entraînement d'agrafe (38) est/sont formé(e)s.

**13.** Instrument médical à tige (1) selon l'une quelconque des revendications 11 ou 12, **caractérisé en ce qu'**une languette (13) est supportée par le rail de transport et d'entraînement (16) et/ou par le rail de retenue (12).

**14.** Instrument médical à tige (1) selon la revendication 13, **caractérisé en ce que** ces pattes élastiques de support (60) font saillie depuis le rail de retenue (12) en direction de la languette (13).

Fig. 1

Fig. 2

EP 3 136 988 B1

Fig. 3

Fig. 5

Fig. 6

Fig. 4

Fig. 7

Fig. 8

Fig. 9

Fig. 10

Fig. 11

Fig. 12

Fig. 13

Fig. 14

Fig. 15

Fig. 16

38

16

12

13

8

## Fig. 17

38    38    38    13    16    38    13    38

32    32    32    32

## Fig. 18

16    19

43    44

13    40

39    16

40    42

41    39

42    43

19

## Fig. 19          ## Fig. 20

Fig. 21

Fig. 22

Fig. 23

Fig. 24

Fig. 25

Fig. 26

Fig. 27

Fig. 28

Fig. 29

Fig. 30

Fig. 31

Fig. 32

60

55

56

12

32

60

Fig. 33

64, 65, 66

13

62, 63

61

68, 69

Fig. 34

Fig. 35

Fig. 36

Fig. 37

Fig. 38

Fig. 39

Fig. 40

Fig. 41

Fig. 42

Fig. 43

**Fig. 44**

**Fig. 45**

**Fig. 46**

**Fig. 47**

**Fig. 48**

**Fig. 49**

Fig. 50

Fig. 51

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0697198 A1
- DE 102009018820 A1
- US 20110224701 A1
- EP 0086721 A2
- EP 0409569 A1
- EP 2609877 A1
- EP 0089737 A1
- WO 03005911 A1
- EP 1712187 A2
- WO 2008127968 A
- US 20050171560 A1